# EUROPÄISCHE PATENTSCHRIFT

(11) **EP 0 454 060 B1**
(45) Veröffentlichungstag und Bekanntmachung des Hinweises auf die Patenterteilung: **03.07.1996**
(21) Anmeldenummer: 91106519.1
(22) Anmeldetag: 23.04.1991
(51) Int. Cl.: C07D 239/96, A61K 31/505

(54) **3-(Mercaptoalkyl)-chinazolin-2,4(1H,3H)-dione, Verfahren zu ihrer Herstellung und pharmazeutische Zubereitungen**
3-(Mercaptoalkyl)-quinazoline-2,4(1H,3H)-diones, processes for their preparation, and pharmaceutical compositions
3-(Mercaptoalkyl)-quinazoline-2,4(1H,3H)-diones, procédés pour leur préparation et compositions pharmaceutiques

(30) Priorität: 24.04.1990 DD 340025; 24.04.1990 DD 340026; 24.04.1990 DD 340027; 24.04.1990 DD 340029; 24.04.1990 DD 340032; 24.04.1990 DD 340035
(43) Veröffentlichungstag der Anmeldung: 30.10.1991
(73) Patentinhaber: Drössler, Karl, Prof. Dr., 04207 Leipzig (DE); Leistner, Siegfried, 04349 Leipzig (DE)
(72) Erfinder: Leistner, Siegfried, Dr. sc., O-7042 Leipzig (DE); Gütschow, Michael, Dr. Dipl.-Bioch., O-7039 Leipzig (DE); Drössler, Karl, Dr. sc., O-7062 Leipzig (DE); Vieweg, Helmut, Dr., W-7888 Rheinfelden (DE); Wagner, Günther, Prof. Dr. sc., O-7027 Leipzig (DE); Strohscheidt, Thomas, O-7010 Leipzig (DE); Lohmann, Dieter, Dr. sc., O-8122 Radebeul (DE); Laban, Gunter, Dr., O-8010 Dresden (DE); Ambrosius, Herwart, Prof. Dr. sc. Dr. h.c., O-7030 Leipzig (DE); Siegling, Angela, Dipl.-Päd., O-9535 Weissbach (DE)
(74) Vertreter: Patentanwälte Beetz - Timpe - Siegfried Schmitt-Fumian - Mayr

(56) Entgegenhaltungen:
- EP-A- 0 219 259
- DE-A- 1 934 037
- DE-A- 3 311 925
- GB-A- 1 063 894
- JOURNAL OF HETEROCYCLIC CHEMISTRY, vol. 7, no. 6, 1970, Seiten 1337 - 1345; R. L. JACOBS: "The Synthesis of o-Amino-N-substituted Benzamides and 3-Substituted 2,4[1H,3H]-Quinazolinediones from Isatoic Anhydride"
- PHARMAZIE, vol. 31, no. 9, 1976, Seiten 601 - 603; M.I. EL-ASHMAWI et al.: "Synthesis of 1.2.3.4-Tetrahydro-2.4-dioxoquinazoline Derivatives for Pharmacological Study"
- HELVETICA CHIMICA ACTA, vol. 50, no. 5, 1967, Seiten 1440 - 1452; E. CHERBULIEZ et al.: "148. Recherches sur la formation et la transformation des esters LXXV[1]. Amines, amino-alcools et aminoalcoyl-monoesters ..."

## Beschreibung

Die Erfindung betrifft 3-(Mercaptoalkyl)-chinazolin-2,4(1H,3H)-dione der allgemeinen Formel I, 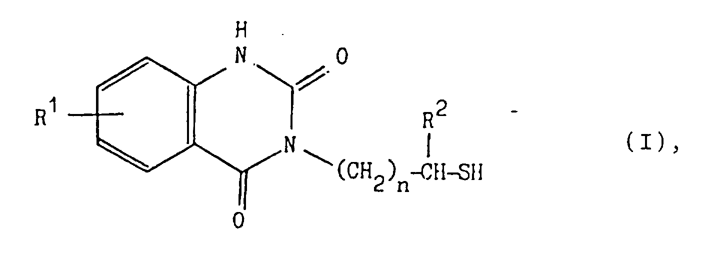 worin bedeuten:
- R¹: Wasserstoff, 6-Methyl, 6-Fluor, 6-Chlor, 6-Brom oder 6,7-Dimethoxy,
- R²: Wasserstoff oder Methyl
und
- n: 1 oder 2,
sowie deren Tautomere und Salze, Verfahren zu ihrer Herstellung und pharmazeutische Zubereitungen, insbesondere zur Behandlung von Immunerkrankungen und/oder Virusinfektionen bei Mensch und Tier.

Verbindungen der allgemeinene Formel I sind in der Fach- und Patentliteratur bislang noch nicht beschrieben.

Es sind bereits Verfahren zur Herstellung von Arzneimitteln mit immunstimulierender und/oder immunrestaurativer Wirkung bekannt, die einen niedermolekularen Wirkstoff enthalten. Als Arzneimittel werden derzeit vor allem Levamisol-hydrochlorid und Inosin-benzoat verwendet.

Andere Wirkstoffe, wie NPT 15 392 (Erythro-9-(2-hydroxy-3-nonyl)-hypoxanthin), Azimexon und Dithiocarbum, weisen ebenfalls immunstimulierende und/oder immunrestaurative Wirkungen auf. Arzneimittel, die diese letztgenannten Wirkstoffe enthalten, sind jedoch bisher nicht eingeführt.

Eine Standardisierung von bekannten und neuartigen Wirkstoffen, die im Sinne einer Stimulierung und/oder Restaurierung auf das Immunsystem wirken, ist derzeit nur schwer möglich. Das ergibt sich einmal aus der Komplexizität des Immunsystems mit seinen vielfältigen Regulations- und Gegenregulationsmechanismen und hängt zum anderen auch mit fehlenden, international verbindlich vereinbarten Versuchsbedingungen bei Labortieren bzw. ebensolchen Bedingungen für klinische Untersuchungen am Menschen zusammen.

Ferner werden die eingangs erwähnten Wirkstoffe hinsichtlich ihres immunstimulierenden und/oder immunrestaurierenden Wirkungsmechanismus weiterhin vertieft untersucht.

Einer umfassenden Vergleichbarkeit immunstimulierend und/oder immunrestaurierend wirkender Verbindungen steht auch die unterschiedliche Beeinflussung der verschiedenen immunkompetenten Zellen durch die betreffenden Wirkstoffe entgegen, woraus sich eine nicht gleichartige Einflußnahme auf spezifische und unspezifische Abwehrmechanismen ergibt.

So wirkt zum Beispiel Levamisol-hydrochlorid vorwiegend immunrestaurierend bei immunsupprimierten Organismen. Bei nur geringem Einfluß auf die humorale Antwort werden vorwiegend T-Zellen stimuliert. Eine Aktivierung unspezifischer Abwehrmechanismen ist durch Stimulierung der Phagozytose sowie der Proliferation und Bakterizidie von Makrophagen gegeben.

Inosin-benzoat dagegen zeigt vor allem eine immunstimulierende Wirkung unter anderem durch Steigerung der humoralen Immunantwort. Die Proliferation und Differenzierung der T-Lymphozyten werden ebenso gesteigert wie die über Lymphokin-Induktion vermittelte Makrophagenfunktion.

Trotz der erzielten Fortschritte auf dem Gebiet der Entwicklung von immunstimulierend und/oder immunrestaurierend wirkenden Substanzen weisen die wenigen bislang bekannten Verfahren zur Bereitstellung derartiger Arzneistoffe Nachteile auf. So ruft das durch Diastereomerentrennung des racemischen Tetramisolhydrochlorids erhältliche optische Isomer Levamisol-hydrochlorid nach oraler Applikation beim Menschen einen bittermetallischen Geschmack hervor und kann zum Erbrechen, Nausea, Leukopenie und Agranulozytose führen. Weiterhin weist Levamisolhydrochlorid eine bemerkenswerte Abhängigkeit in der Wirkung von genetischen Faktoren, Alter und Geschlecht der Patienten auf.

Das in relativ hohen Dosen zu verabreichende Inosin-benzoat (50 mg/kg·d) kann zum Erbrechen, Hyperurikämie und Haematokritanstieg führen. Beim Azimexon werden Kopfschmerz, Erbrechen, Hb- und Erythrozyten-Abnahme als Nebenwirkung beobachtet. Gründe für die genannten Nachteile sind in der Art der bisher verwendeten Wirkstoffe zu suchen.

Es besteht daher ein Bedürfnis, Arzneimittel zur Behandlung von Immunerkrankungen und/oder Virusinfektionen bei Mensch und Tier sowie Verfahren zu ihrer Herstellung zu entwickeln. Insbesondere besteht ein Bedürfnis nach neuen Arzneimitteln mit immunstimulierender und/oder immunrestaurativer Wirkung für Mensch und Tier, die zu einer erhöhten spezifischen und unspezifischen Abwehrlage führen.

Die Erfindung hat die Aufgabe, neue chemische Substanzen aufzufinden, die immunstimulierende und/oder immunrestaurative und/oder antivirale Wirkungen besitzen, wenn sie dem menschlichen oder einem tierischen Körper zugeführt werden. Es ist weiter die Aufgabe dieser Erfindung, Verfahren zur Herstellung solcher Verbindungen als auch entsprechender Arzneimittel zu entwickeln, welche diese Verbindungen enthalten.

Die Aufgabe wird anspruchsgemäß gelöst. Die abhängigen Ansprüche betreffen vorteilhafte Ausführungsformen der Erfindung.

Die erfindungsgemäßen 3-(Mercaptoalkyl)-chinazolin-2,4(1H,3H)-dione besitzen die allgemeine Formel I, 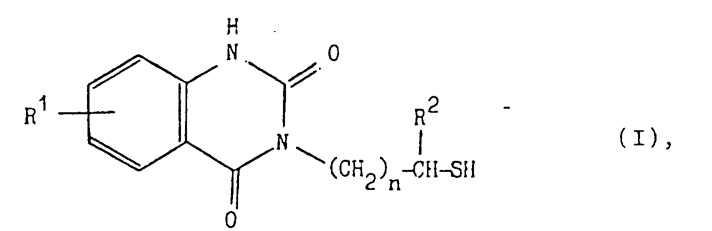 worin bedeuten:
- R¹: Wasserstoff, 6-Methyl, 6-Fluor, 6-Chlor, 6-Brom oder 6,7-Dimethoxy,
- R²: Wasserstoff oder Methyl
und
- n: 1 oder 2.

Unter den erfindungsgemäßen Verbindungen werden auch ihre Salze, insbesondere ihre pharmazeutisch geeigneten Salze verstanden, besonders die Alkali- und Ammoniumsalze, sowie auch ihre Tautomeren.

Die Verbindungen der Formel I sind neue chemische Substanzen mit immunstimulierenden und/oder immunrestaurativen und/oder antiviralen Wirkungen, aufgrund deren sie in der Human- und Veterinärmedizin vorteilhaft einsetzbar sind.

Von den Verbindungen der allgemeinen Formel I sind als besondere Ausführungsform der Erfindung die Verbindungen der allgemeinen Formel II hervorzuheben, 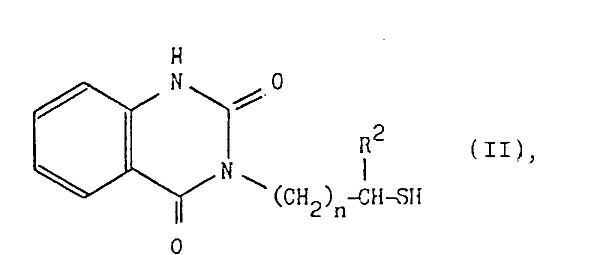 worin R² und n die oben genannten Bedeutungen besitzen,
und hierunter insbesondere das 3-(Mercaptoethyl)-chinazolin-2,4(1H,3H)-dion.

Entsprechend der vorliegenden Erfindung können die Verbindungen der allgemeinen Formel I sowohl als solche als auch in ihrer tautomeren Form vorliegen, ferner auch als Salze, insbesondere als pharmazeutisch unbedenkliche Alkali- oder Ammoniumsalze.

Die erfindungsgemäßen Verfahren zur Herstellung der Verbindungen der allgemeinen Formeln I und II sind gekennzeichnet durch folgende Verfahrensweisen:
(A) Umsetzung von 2H-3,1-Benzoxazin-2,4(1H)-dionen der allgemeinen Formel III, 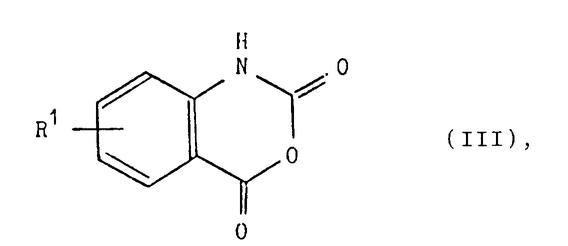 worin R¹ dasselbe wie oben bedeutet,
   mit einem Aminoalkanol der allgemeinen Formel IV, 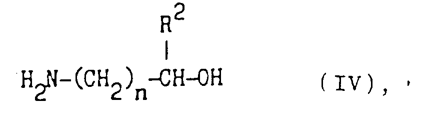 worin R² und n dasselbe wie oben bedeuten,
   vorzugsweise in einem wäßrigen Reaktionsmilieu, zu einer Verbindung der allgemeinen Formel V, 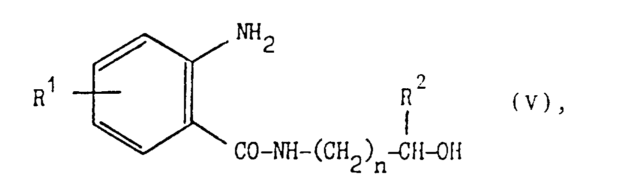 worin R¹, R² und n dasselbe wie oben bedeuten,
   - Zugabe einer Reaktionslösung aus einem C₁₋₃-Alkanol, Schwefelkohlenstoff und Natrium- oder Kaliumhydroxid bzw. Natrium- oder Kaliumxanthogenat,
   - Erhitzen des Reaktionsgemisches, vorzugsweise bis zum Rückfluß,
   - Abkühlen und Ansäuern des Reaktionsgemisches,
   - Erhitzen der erhaltenen Verbindungen der allgemeinen Formel VI, 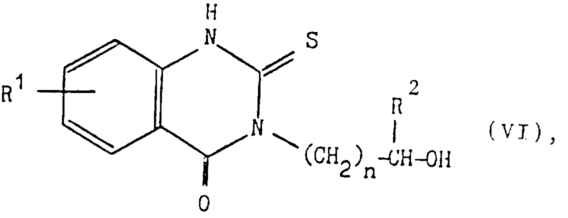 worin R¹, R² und n dasselbe wie oben bedeuten,
      mit konzentrierter Mineralsäure, wie Salzsäure und/oder Schwefelsäure, oder Mischungen dieser Mineralsäuren mit Eisessig und/oder Ameisensäure,
   - und ggfs. anschließende Zugabe von Wasser zum Reaktionsansatz und erneutes Erhitzen unter Rückfluß unter Erhalt der Verbindungen der allgemeinen Formel I oder ihrer Tautomeren
   oder
(B)
   - Erhitzen von 4H-3,1-Benzothiazin-2,4(1H)-dithionen der allgemeinen Formel VII, 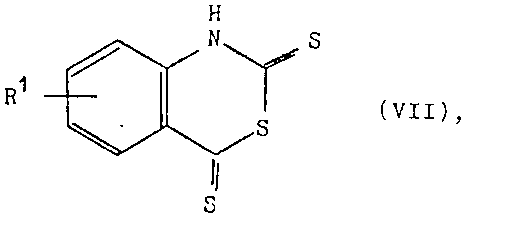 worin R¹ dasselbe wie oben bedeutet,
      mit einem Aminoalkanol der allgemeinen Formel IV, 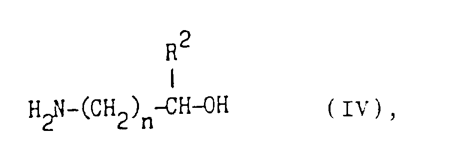 worin R² und n dasselbe wie oben bedeuten,
      in einem polaren organischen Lösungsmittel unter Erhalt der Verbindungen der allgemeinen Formel VIII, 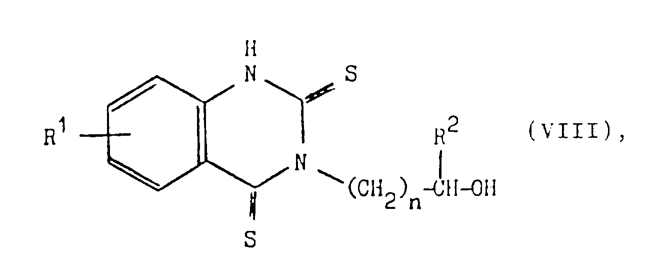 worin R¹, R² und n dasselbe wie oben bedeuten,
   - Umsetzung der Verbindungen der Formel VIII in einem wäßrig-alkanolischen Reaktionsmilieu in Gegenwart eines Säureakzeptors, wie eines Alkalihydroxids oder Triethylamin, mit einem Alkylhalogenid der allgemeinen Formel IX,

      R³-X (IX),

      worin
      R³ C₁₋₃-Alkyl und
      X Jod oder Brom
      bedeuten,
      vorzugsweise bei Raumtemperatur,
      zu den Verbindungen der allgemeinen Formel X, 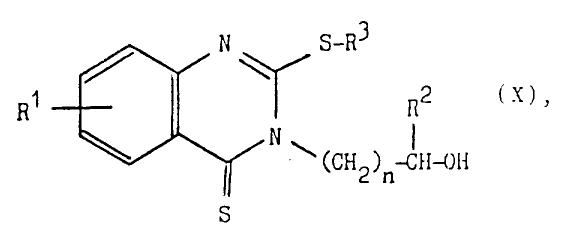 worin R¹, R², R³ und n dasselbe wie oben bedeuten,
   - Umsetzung der Verbindungen der Formel X mit alka nolischer Mineralsäure, vorzugsweise absoluter ethanolischer Salzsäure, zu tricyclischen Chinazoliniumsalzen der allgemeinen Formel XI, 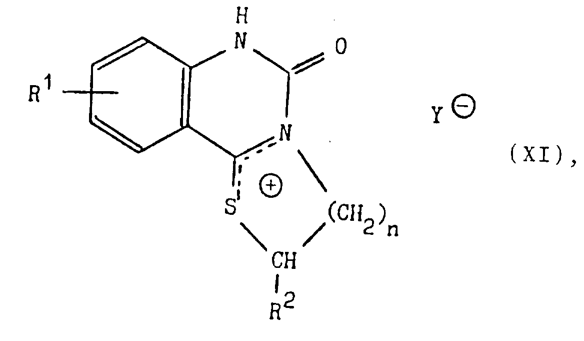 worin
      R¹, R² und n dasselbe wie oben bedeuten und
      Y ein Säureanion, vorzugsweise Chlor, darstellt,
      und
   - Überführung der Verbindungen der Formel XI durch Behandlung in alkanolisch-wäßriger Natronlauge, nachfolgende Filtration und Ansäuern des Filtrats mit verdünnter Mineralsäure in die Verbindungen der allgemeinen Formel I oder ihre Tautomeren
   oder
(C)
   - Umsetzung von 2H-Benzoxazin-2,4(1H)-dionen der allgemeinen Formel III, 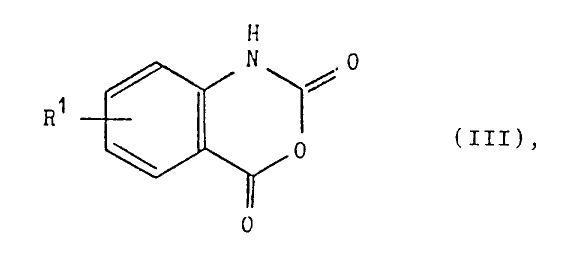 worin R¹ dasselbe wie oben bedeutet,
      mit Bis(aminoalkyl)-disulfanen der allgemeinen Formel XII, 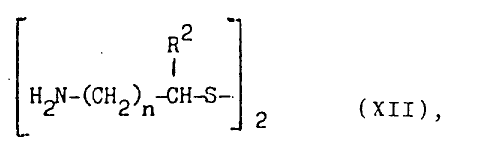 worin R² und n dasselbe wie oben bedeuten,
      oder deren Salzen, vorzugsweise den Dihydrochloriden, in einem Lösungsmittel zu Verbindungen der allgemeinen Formel XIII, 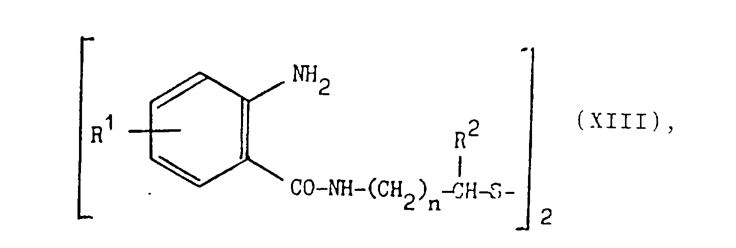 worin R¹, R² und n dasselbe wie oben bedeuten,
   - Umsetzung der Verbindungen der Formel XIII mit Chlorameisensäurealkylestern der allgemeinen Formel XIV,

      Cl-COOR⁴ (XIV),

      worin R⁴ C₁₋₃-Alkyl bedeutet,
      in einem Lösungsmittel oder Lösungsmittelgemisch zu Verbindungen der allgemeinen Formel XV, 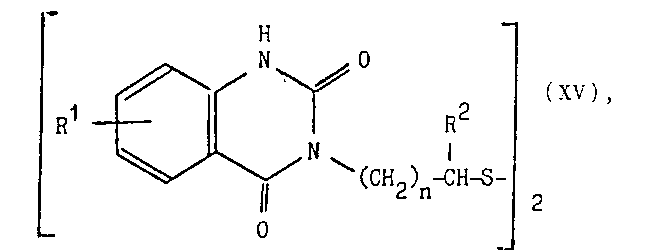 worin R¹, R² und n dasselbe wie oben bedeuten,
      und
   - Überführung der Verbindungen der Formel XV, gegebenenfalls nach Reinigung und Trocknung, in einem Lösungsmittel oder Lösungsmittelgemisch mit nascierendem Wasserstoff in die Verbindungen der allgemeinen Formel I oder ihre Tautomeren
   oder
(D)
   - Umsetzung von 3-(Haloalkyl)-2,4-dioxo-1,2,3- tetrahydrochinazolinen der allgemeinen Formel XVI, 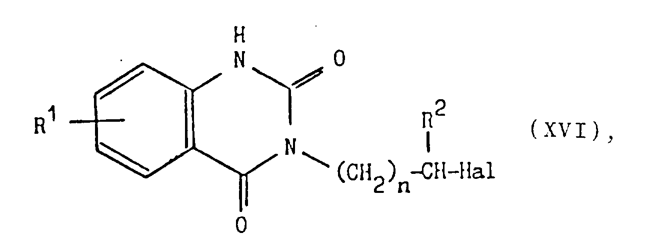 worin
      R¹, R² und n dasselbe wie oben bedeuten und
      Hal Chlor oder Brom darstellt,
      mit Thioharnstoff, vorzugsweise im Temperaturbereich von 80 bis 150 °C, zu den S-[ω-(2,4-Dioxo-1,2,3,4-tetrahydrochinazolin-3-yl)-alkyl]-isothiuroniumhalogeniden der allgemeinen Formel XVII, 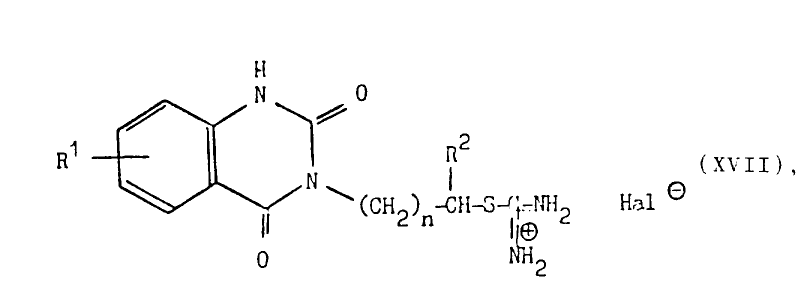 worin R¹, R², n und Hal dasselbe wie oben bedeuten,
      und
   - Überführung der Verbindungen der Formel XVII, gegebenenfalls nach ihrer Abtrennung, mit Alkalilauge in die Verbindungen der allgemeinen Formel I oder ihre Tautomeren,
      vorzugsweise in Gegenwart eines Schutzgases, wie Stickstoff, und vorteilhaft im Temperaturbereich von -10 bis +15 °C,
   sowie gegebenenfalls Überführung der nach den Verfahrensweisen A-D erhaltenen Verbindungen der allgemeinen Formel I in Salze, vorzugsweise in pharmazeutisch unbedenkliche Salze, wie die Alkalisalze oder Ammoniumsalze.

Entsprechend der vorliegenden Erfindung können die unter (A) bis (D) beschriebenen Verfahren in weiten Grenzen variiert werden.

So können die nach dem Verfahren (A) aus den Umsetzungen der Verbindungen der allgemeinen Formel VI mit konzentrierten Mineralsäuren, wie Salzsäure oder Schwefelsäure, oder Mischungen dieser Mineralsäuren mit Eisessig und/oder Ameisensäure, resultierenden Feststoffgemische abgetrennt und nachfolgend durch Erhitzen in verdünnten Mineralsäuren in die Verbindungen der allgemeinen Formel I oder deren Tautomere übergeführt werden.

Weitere Ausführungsformen der Erfindung können beispielsweise bei der Durchführung des Verfahrens (D) darin bestehen,
- daß die Umsetzung der Verbindungen der allgemeinen Formel XVI mit Thioharnstoff in einem Molverhältnis von 1:1 bis 1:3 erfolgt,
- daß zur Herstellung der Verbindungen der allgemeinen Formel XVII die Reaktionspartner in einem protischen Lösungsmittel, vorzugsweise Methylglykol oder Butan-1-ol, vorzugsweise unter Rückfluß, umgesetzt werden,
- daß bei der Umsetzung der Verbindungen der allgemeinen Formel XVI, insbesondere, wenn diese reaktionsträge sind, im Vakuum gearbeitet wird,
- daß die Umsetzung der Verbindungen der allgemeinen Formel XVII zu den Verbindungen der allgemeinen Formel I in wäßriger oder wäßrig-ethanolischer Alkalilauge, insbesondere Natronlauge mit einer Konzentration von 0,1 bis 1,0 mol/l und vorzugsweise 0,25 mol/l, und gegebenenfalls unter Rühren erfolgt, und/oder
- daß die Umsetzung der Verbindungen der allgemeinen Formel XVII zu den Verbindungen der allgemeinen Formel I unter einer Schutzgas-Atmosphäre, vorteilhaft unter Stickstoff, durchgeführt wird, was auch bei der Umkristallisation der Verbindungen der allgemeinen Formel I vorteilhaft ist.

In einer weiteren Ausführungsform der Erfindung kann die Umsetzung der Verbindungen der allgemeinen Formel XVI zu den Verbindungen der allgemeinen Formel I auch so erfolgen, daß auf die Abtrennung der Verbindungen der allgemeinen Formel XVII verzichtet wird, ansonsten jedoch die oben genannten Reaktionsbedingungen, sinngemäß angewandt, eingehalten werden.

Die Verbindungen der allgemeinen Formel I können entsprechend einer weiteren Ausführungsform der Erfindung in an sich bekannter Weise in ihre Salze, vorteilhaft die Alkali- oder Ammoniumsalze, übergeführt werden. Ein weiterer Aspekt der vorliegenden Erfindung besteht in pharmazeutischen Zubereitungen zur Behandlung von Immunerkrankungen und/oder Virusinfektionen bei Mensch und Tier, welche eine oder mehrere Verbindungen der Formel I und/oder deren Tautomere und/oder deren pharmazeutisch unbedenkliche Salze, vorteilhaft die Alkali- oder Ammoniumsalze, und inerte, pharmazeutisch übliche Hilfs- und/oder Trägerstoffe enthalten.

Aufgrund der immunregulatorischen, immunstimulierenden und immunrestaurativen Eigenschaften der Verbindungen der allgemeinen Formel I sind derartige pharmazeutische Zubereitungen, welche diese Verbindungen enthalten, insbesondere zur Behandlung von Immunerkrankungen bei Mensch und Tier geeignet.

Aufgrund der gleichzeitig vorhandenen antiviralen Wirkung der Verbindungen der allgemeinen Formel I sind derartige pharmazeutische Zubereitungen, welche diese Verbindungen enthalten, gleichzeitig zur Behandlung von Viruserkrankungen bei Mensch und Tier geeignet.

Eine besondere Ausführungsform der Erfindung besteht darin, daß die o.g. pharmazeutischen Zubereitungen insbesondere Verbindungen der allgemeinen Formel II wie oben definiert und/oder ihre Tautomere und/oder ihre pharmazeutisch unbedenklichen Salze, besonders die Alkali- oder Ammoniumsalze, enthalten.

Ganz besonders bevorzugt sind solche pharmazeutischen Zubereitungen, welche als wirksamen Bestandteil die Verbindung 3-(2-Mercaptoethyl)-chinazolin-2,4(1H,3H)-dion, ihr Tautomeres und/oder pharmazeutisch unbedenkliche Salze dieser Verbindungen, besonders die Alkali- oder Ammoniumsalze, enthalten.

Cherbuliez et al. (Helv. chim. Acta 50 (1967) 1440 - 1452) haben drei Verbindungen, die unter die Definition der allgemeinen Formel VI dieser Erfindung fallen, zweistufig zu 2-(o-Carboxyphenylamino)-4,5-dihydrothiazolen beziehungsweise 5,6-dihydro-4H-1,3-thiazin umgesetzt und strukturanalytisch (Elementaranalyse, ¹H-NMR, UV, IR) untersucht.

### Pharmakologische Versuche

Wie bereits ausgeführt, besitzen die Verbindungen der allgemeinen Formel I wertvolle pharmakologische Eigenschaften, insbesondere zur Behandlung von Immunerkrankungen und/oder Virusinfektionen bei Mensch und Tier.

Im folgenden sind die Ergebnisse über
- immunregulatorische,
- immunstimulierende,
- immunrestaurative und
- antivirale
Eigenschaften der erfindungsgemäßen Verbindungen aufgeführt.

Der Nachweis einer immunregulatorischen Wirksamkeit wurde am Meerschweinchen (Albino-Koloniezucht) und u. a. mit Hilfe folgender Techniken geführt:
- Perkutaner Test zum Nachweis einer Kontaktüberempfindlichkeit vom verzögerten Typ (Effektor-T-Lymphozyten-Aktivität),
- Takatsy-Test zum Nachweis DNP-spezifischer Serumantikörper (humorale Immunantwort).

Der Nachweis immunstimulierender Effekte wurde weiterhin an der Maus (Inzuchtstamm CBA) und u. a. mit Hilfe folgender Nachweismethoden geführt:
- Plaque-Test zum Nachweis von SE-spezifischen IgM- und IgG-PBZ,
- Rosettentest.

Der Nachweis von immunrestaurativen Wirkungen erfolgte nach Schädigung des Immunsystems ebenfalls mittels
- Plaque-Test zum Nachweis von SE-spezifischen IgM- und IgG-PBZ sowie durch
- Rosettentest.

Der Nachweis der antiviralen Wirksamkeit erfolgte in vitro an Hühnerembryonalzellen mit Vertretern verschiedener Virusgruppen.
1. Untersuchungen zur immunregulatorischen Wirksamkeit
1.1 Perkutaner Test zum Nachweis einer Kontaktüberempfindlichkeit
Die epikutane Applikation von Dinitrofluorbenzol (DNFB) führt beim Meerschweinchen
und bei der Maus zur Entwicklung einer verzögerten Überempfindlichkeit, eines Immunstatus, der an die klonale Vermehrung TNP-spezifischer T-Lymphozyten (T_{DTH}-Ly) gebunden ist. Erfolgt nach einer Latenzperiode von mindestens 5 Tagen eine erneute DNFB-Applikation, kommt es zu einer Freisetzung von Lymphokinen durch Reaktion mit den T_{DTH}-Ly dieses Hautbereiches und dadurch zu einer lokal begrenzten Entzündungsreaktion.
Sieben Tage nach der Immunisierung mit 1 %igem DNFB wurden die Flanken der Versuchstiere enthaart und durch Aufbringen je eines Tropfens 0,5 %, 0,1 %, 0,05 %, 0,025 % und 0,01 %iger DNFB-Lösung getestet. Die noch positive Konzentration ist dem Grad der Sensibilisierung umgekehrt proportional. Die Testergebnisse ergeben sich aus Stärke und Anzahl der positiven Hautreaktionen, der Stimulierungsindex (SI) aus dem Vergleich der Test- und Kontrolltiere.
Die Substanzbehandlung erfolgte peroral. Mittels Schlundsonde wurde eine tägliche Dosis von 2 mg/kg Körpermasse als Suspension in Wasser gegeben. Behandlungsdauer: 1 Tag + 1 (ein Tag nach der Immunisierung) bis +6. Die Kontrolltiere erhielten nur die eingesetzte Lactose.
1.2 Takatsy-Test
Nach Immunisierung mit Dinitrofluorbenzol (DNFB) kommt es beim Meerschweinchen auch zur Bildung DNP-spezifischer humoraler Antikörper, die bevorzugt der IgG₁-Substanzklasse angehören. 14 Tage nach primärer epikutaner Applikation von DNFB wurde den Versuchstieren Blut durch Herzpunktion entnommen. Die Bestimmung der Antikörperkonzentration erfolgte nach einer von Takatsy beschriebenen Methode (Takatsy, G.: A new method for the preparation of serial dilutions in a quick and accurate way. Kiserletes Orvostudomany 2 (1950) 263 - 396). Als Indikatorzellen wurden TNP-beladene Schaferythrozyten verwendet (Kopplung nach Rittenberg und Pratt; Rittenberg, M. B. and Pratt, K. L.: Antitrinitrophenyl (TNP) plaque assay. Primary response of BALB/c mice to soluble and particulate immunogen. Proc. Soc. exp. Biol. Med., 132 (1969) 575 - 579).
1.3 Ergebnisse
Sensibilisierungsgrad (%) und Serumantikörperkonzentration (AK-Titer-Differenz) gegenüber der jeweiligen Kontrollgruppe (Screening-Werte)

| Verbindung gemäß Beispiel | Kontaktdermatitis (Kontrolle: 100) | | DNP-spezifische Antikörper |
|---|---|---|---|
| | 24-Stunden-Reaktion | 48-Stunden-Reaktion | |
| 6 | 70 | 40 | - 1,5 |
| 7 | 230 | | + 1,8 |
| 12 | 120 | 130 | + 0,3 |
| 13 | 120 | 130 | ± 0 |
| 8 | 100 | 115 | ± 0 |
| 5 | 150 | 120 | - 0,6 |
| 3 | 100 | | + 1,0 |
| 2 | 180 | | ± 0 |
| 14a | 60 | 60 | - 1,9 |
| 14b | 105 | 100 | + 2,2 |
| 14c | 90 | 105 | ± 0 |
| 14d | 100 | 150 | ± 0 |
| 14e | 100 | | ± 0 |

Aus den Werten wird deutlich, daß die Verbindungen der allgemeinen Formel I einen gut nachweisbaren Effekt auf die Effektor-T-Lymphozyten-abhängige verzögerte Uberempfindlichkeit haben. Kontaktdermatitis-Werte ab 120 (≙ 20 % Steigerung gegenüber der Kontrolle) bzw. unter 80 werden als Nachweis eines stimulatorischen bzw. suppressorischen Effektes angesehen.
Antikörpertiter-Differenzen von >+1 bzw. <-1 verdeutlichen den stimulatorischen bzw. suppressorischen Effekt der Substanzen auf die humorale Immunantwort.
2. Untersuchungen zur immunstimulierenden Wirkung
2.1 Plaque-Test zum Nachweis von SE-spezifischen IgM- und IgG-PBZ
   Zur Prüfung immunstimulierender Effekte wurde u. a. auch der Einfluß von Verbindungen der allgemeinen Formel I auf die humorale Immunantwort an Mäusen des Inzuchtstammes CBA und mittels Hämolyse-Plaque-Test (HPT) untersucht. Diese Technik erlaubt die quantitative und getrennte Erfassung IgM- und IgG-Antikörper produzierender Zellen (= Plaque bildende Zellen; PBZ). Die Immunisierung mit Schaferythrozyten (SE) erfolgte am Tag 0, die Substanzapplikation (Tagesdosis 1.10⁻⁵ mol/kg Körpermasse; peroral) an den Tagen -1 bis +3 (zur Erfassung der IgM-PBZ) bzw. an den Tagen +1 bis +5 (zur Erfassung der IgG-PBZ).
2.2 Ergebnisse:
   Durch tägliche und über 5 Tage gehende Gabe von 2,0 mg der Verbindung gemäß Beispiel 1 pro kg Körpermasse konnte die Zahl der IgM-PBZ auf einen Durchschnittswert von 352 % im Vergleich zur Kontrolle (100 %) gesteigert werden. Die gleiche Form der Behandlung führte zu einer Erhöhung der IgG-PBZ auf 497 % (Kontrolle: 100 %). In beiden Fällen erwies sich die Differenz zur Kontrolle im t-Test mit p < 0,001 als signifikant. Es wird deutlich, daß eine über 5 Tage gehende tägliche Behandlung mit der Verbindung gemäß Beispiel 1 bei der CBA-Maus eine hochsignifikante Steigerung SE-spezifischer IgM- und IgG-PBZ hervorruft.
2.3 Rosettentest (SE-spezifische Rosetten)
   Nach der BURNET'schen Theorie tragen die Lymphozyten auf ihrer Oberfläche Immunglobulin-Rezeptoren, die gegen ein bestimmtes Antigen gerichtet und durch eine Reaktion mit dem spezifischen Antigen nachweisbar sind. Die Bestimmung der Anzahl an rosettenbildenden Zellen (RBZ) kann als Maß für die Stärke einer anlaufenden Immunreaktion dienen und folgend auch Effekte im Sinne einer Suppression oder Stimulierung zellproliferativer Prozesse wiedergeben.
   Drei Tage nach der intravenösen Immunisierung mit 2·10⁵ Schaferythrozyten (SE) in 0,2 ml physiologischer Kochsalzlösung wurden die Versuchstiere durch Überstrecken getötet, die Milz entnommen und unter Zugabe von Parker-Medium (pH 7,4) durch Müller-Gaze gerieben. Die Arbeiten zur Gewinnung der Zellsuspension wurden im Eiswasserbad (ca 0 bis 4 °C) durchgeführt. In einer Zählkammer nach Neubauer erfolgte die Bestimmung der Zelldichte, und anschließend wurde die für alle Versuchsansätze konstante Zellzahl von 1·10⁷ kernhaltigen Zellen (KHZ)/ml Medium eingestellt. 0,9 ml der Milzzellsuspension (1·10⁷ KHZ/ml) wurden dann mit 0,1 ml einer 5 %igen gewaschenen Schaferythrozytensuspension versetzt und 24 Stunden bei 4 °C inkubiert. Als Rosette wurde gewertet, wenn eine KHZ von mindestens 4 Erythrozyten umgeben war und die kernhaltige Zelle zu identifizieren war.
2.4 Ergebnisse
   Nach oraler Gabe von Verbindungen gemäß der Beispiele 2, 6 und 14e konnte eine signifikante Förderung SE-spezifischer rosettenbildender Zellen festgestellt werden.

3. Untersuchungen zu immunrestaurativen Effekten
3.1 Der Nachweis der immunrestaurativen Wirksamkeit von Verbindungen der allgemeinen Formel I wurde u. a. dadurch geführt,daß das Immunsystem der Versuchstiere (CBA-Inzuchtmäuse) durch Chemotherapie (Zyklophosphamid, Carageenan, Iloprost) bzw. Bestrahlung geschädigt wurde. Die Prüfung erfolgte u. a. mittels folgender oben erläuterter Techniken:
- Plaque-Test zum Nachweis von SE-spezifischen IgM- und IgG-PBZ,
- Rosettentest.

3.2 Ergebnisse
Der Nachweis immunrestaurativer Effekte der Verbindung gemäß Beispiel 1 wurde u. a. dadurch erbracht, daß das Immunsystem der Versuchstiere durch Zyklophosphamid (ZY) geschädigt wurde und die Prüfung anhand von SE-spezifischer PBZ erfolgte.
Die Schädigung wurde durch Injektion von 150 mg/kg Körpermasse am Tag vor der Immunisierung (-1) vorgenommen (Test- und Kontrollgruppe). Die Imunisierung erfolgte durch intraperitoneale Injektion von 4·10⁸ SE in 0,2 ml PBS.
Die Versuchstiere der Testgruppe erhielten je 2 mg der Verbindung gemäß Beispiel 1 pro kg Körpermasse an den Tagen -2, -1, 0, +1 und +2 durch orale Gabe. Mittels Plaque-Test wurden die SE-spezifischen IgM- und IgG-PBZ nachgewiesen.

**Tabelle:**

| Resultate (IgM-PBZ) | | |
|---|---|---|
| Tag der Testung | IgM-PBZ/10⁶ | |
| | Kontrolle Nur ZY, keine Verbindung gemäß Beispiel 1 | Test ZY + Verbindung gemäß Beispiel 1 |
| + 3 | 37,5 ± 27,1 | 31,7 ± 26,6 |
| + 4 | 49,7 ± 32,9 | 59,8 ± 26,6 |
| + 5 | 56,8 ± 27,6 | 125,2 ± 32,7 |
| + 6 | 74,7 ± 73,4 | 47,8 ± 31,2 |

Unter dem Einfluß der Verbindung gemäß Beispiel 1 kommt es zu einer meßbaren Erholung der immunologischen Reaktivität, die am Tag +5 mit p <0,05 signifikant ist.

**Tabelle:**

| Resultate (IgG-PBZ) | | |
|---|---|---|
| Tag der Testung | IgG-PBZ/10⁶ Milzzellen | |
| | Kontrolle nur ZY, keine Verbindung gemäß Beispiel 1 | Test ZY + Verbindung gemäß Beispiel 1 |
| + 6 | 26,7 ± 12,1 | 88,3 ± 24,8 |
| + 7 | 46,7 ± 16,3 | 128,3 ± 28,6 |
| + 8 | 63,3 ± 24,2 | 58,3 ± 43,6 |

Die Verbindung gemäß Beispiel 1 steigert die Anzahl der IgG-PBZ bei ZY-supprimierten Versuchstieren an den Tagen 6 + 7 signifikant (p <0,05 bzw. p <0,01).
4. Untersuchungen zur antiviralen Wirksamkeit
Der Nachweis der antihumanviralen und antizooviralen Wirkung erfolgte nach Auflösen der Testsubstanz in Dimethylsulfoxid (50 mmol/l) und nachfolgender Verdünnung der Stammlösung 1 : 200 bis 1 : 1600 mit üblichen physiologischen Medien. Bei dieser Verfahrensweise konnte eine unspezifische Wirkung des organischen Lösungsmittels vermieden werden.
Die antivirale Testung erfolgte invitro an Hühnerembryonalzellen mit Vertretern der Virusgruppen Pox- Influenza-, Herpes simplex- und Rhabdoviren sowie MDBK-Zellen (Rindernierenzellen), humane Fibroplasten/Rhabdo- und Coxsackieviren wie auch RH (Zellen) (menschliche Nierenzellen) und Pox-, Herpes simplex- und Adenoviren
- in Einstufenreplikationsversuchen nach Tonew und Tonew (Arch. ges. Virusforsch. 33, 319 - 329, 1971),
- Plaquereduktionstests nach Tonew und Tonew (Zbl. Bakter. Hyg. I. Orig. 211, 437 - 444, 1969) wie auch
- im Mikrotitersystem Dynatech nach Tonew und Glück (J. basic Microbiol. 1986, (3) 173).

Die Untersuchung der Zellverträglichkeit der Substanz wurde mit denselben Zellen durchgeführt (s. Beispielreihen 1. 2, 3, 4 und 5). Wie die Beispielreihen 1 bis 5 zeigen, sind die getesteten Verbindungen der allgemeinen Formel in therapeutischen Konzentrationen zellverträglich und hemmen eine Reihe von Human- und Zooviren außerordentlich stark.

### Beispielreihe 1

Bestimmungen der Zellverträglichkeit von 3-(2-Mercaptoethyl)chinazolin-2,4(1H,3H)-dion auf primären Hühnerembryonalzellen (2 Tage alt), RH (humane Nierenzellen-Permanentzellinie), MDBK (bovine Nierenzellen-Permanentzellinie) und Human-Fibroblasten (2 Tage alt)

| Zellen | Konzentration der Substanz in µmol/l im Erhaltungsmedium | | | | |
|---|---|---|---|---|---|
| | 1000 | 500 | 250 | 125 | 62,5 |
| HEZ | +++ | + | 0̸ | 0̸ | 0̸ |
| RH | +++ | ++ | 0̸ | 0̸ | 0̸ |
| MDKB | ++++ | ++ | 0̸ | 0̸ | 0̸ |
| HFi | ++ | 0̸ | 0̸ | 0̸ | 0̸ |

- 0̸ =: Am 5. Tag der mikroskopischen Ablesung keine morphologischen Veränderungen der Zellen.
- ++++ =: Komplette Veränderungen der Zellschicht in Form von Abrundung, Granulierung und Ablösen der Zellen von der Glaswand.
- +++ =: Analoge Veränderungen bei 3/4 des Zellrasens.
- ++ =: Analoge Veränderungen bei 1/2 des Zellrasens.
- + =: Analoge Veränderungen bei 1/4 des Zellrasens.

### Beispielreihe 2

Ergebnisse antiviraler Wirkung von 3-(2-Mercaptoethyl)-chinazolin-2,4(1H,3H)-dion auf Hühnerembryonalzellen (HEZ) im Einstufenreplikationszyklus

| Virusstamm | Konzentration in µmol/l | Senkung des Virusertrages im Vergleich zur unbehandelten Kontrolle in log₁₀ TCID₅₀/0,2 ml | % Hemmung |
|---|---|---|---|
| Vaccinia | 250 | 5,67 | > 99,99 |
| Lister | 125 | 4,0 | 99,99 |
| | 62,5 | 2,5 | 99,68 |
| | 31,25 | 1,0 | 99,0 |
| Influenza | 250 | 6,23 | > 99,99 |
| A/WSN | 125 | 4,67 | > 99,99 |
| | 62,5 | 4,25 | > 99,99 |
| | 31,25 | 2,5 | 99,50 |
| Herpes | 250 | 1,0 | 99 |
| simplex Typ 1 | 125 | 0,33 | n. s. |
| Vesicular- | 250 | 3,0 | 99,9 |
| stomatitis | 125 | 0,5 | n. s. |
| Indiana | | | |

Die Ergebnisse sind Mittelwerte aus drei Versuchen. Gegenüber Coxsackie A 9-Virus und Adeno-Virus Typ 4 erwies sich die Substanz als nicht antiviral wirksam.

Therapeutischer Index = Verhältnis von maximal verträglicher und minimal wirksamer Dosis:
für Vaccinia-Virus = 8
für Influenza-Virus = 8 n.s. = nicht signifikant.

### Beispielreihe 3

Ergebnisse antiviraler Wirkung von 3-(2-Mercapto ethyl)-chinazolin-2,4(1H,3H)-dion auf die humane Nierenzellen-Permanentzellinie im Einstufenreplikationszyklus

| Virusstamm | Konzentration in µmol/l | Senkung des Virusertrages im Vergleich zur unbehandelten Kontrolle in log₁₀ TCID₅₀/0,2 ml | % Hemmung |
|---|---|---|---|
| Vaccinia | 250 | > 4,0 | > 99,99 |
| Lister | 125 | > 4,0 | > 99,99 |
| | 62,5 | 4,0 | 99,99 |
| | 31,25 | 3,5 | > 99,9 |
| | | | |
| Herpes | 250 | 6,0 | > 99,99 |
| simplex | 125 | 5,5 | > 99,99 |
| Typ 1 Kupka | 62,5 | 0,75 | n. s. |
| Die Ergebnisse sind Mittelwerte aus zwei Versuchen. n. s. = nicht signifikant | | | |

### Beispielreihe 4

Ergebnisse der antiviralen Wirkung von 3-(2-Mercaptoethyl)chinazolin-2,4(1H,3H)-dion im Plaquereduktionstest

| Virusstamm | Konzentration in µmol/l | Plaquereduktion in % zur Viruskontrolle auf primären HEZ |
|---|---|---|
| Vaccinia | 250 | 100 |
| Lister | 125 | 96 |
| | 62,5 | 92 |
| | 31,25 | 87 |
| | | |
| Influenza | 250 | 100 |
| A/WSN | 125 | 98 |
| | 62,5 | 92 |
| | 31,25 | 85 |
| | | |
| Vesicular- | 250 | 45 ^{x} |
| stomatitis | 125 | 15 |
| Indiana | 62,5 | 0 |
| Die Ergebnisse sind Mittelwerte aus zwei Versuchen. | | |

| | | |
|---|---|---|
| x = keine signifikante Wirkung. | | |

### Beispielreihe 5a

Ergebnisse antiviraler Wirkung von 3-(3-Mercapto prop-1-yl)-6,7-dimethoxychinazolin-2,4(1H,3H)-dion auf humane Nierenzellen-Permanentzellinie im Einstufenreplikationszyklus

| Virusstamm | Konzentration in µmol/l | Senkung des Virusertrages im Vergleich zur unbehandelten Kontrolle in log₁₀ TCID₅₀/0,2 ml | % Hemmung |
|---|---|---|---|
| Vaccinia | 250 | 5,0 | > 99,99 |
| Lister | 125 | 5,0 | > 99,99 |
| | 62,5 | 5,0 | > 99,99 |
| | 31,25 | 2,84 | 99,84 |
| | | | |
| Herpes | 250 | 6,5 | > 99,99 |
| simplex | 125 | 6,5 | > 99,99 |
| Typ 1 | 62,5 | 5,0 | > 99,99 |
| | 31,25 | 3,67 | 99,97 |
| | | | |
| Influenza | 250 | 6,17 | 99,99 |
| A/WSN | 125 | 2,0 | 99,0 |
| | 62,5 | 0,5 | n. s. |
| | 31,25 | 0 | |
| n. s. = nicht signifikant | | | |

### Beispielreihe 5b

Ergebnisse antiviraler Wirkung von 3-(3-Mercaptoprop-1-yl)-6,7-dimethoxychinazolin-2,4(1H,3H)dion auf Hühnerembryonalfibroblasten im Einstufenreplikationszyklus

| Virusstamm | Konzentration in µmol/l | Senkung des Virusertrages im Vergleich zur unbehandelten Kontrolle in log₁₀ TCID₅₀/02 ml | % Hemmung |
|---|---|---|---|
| Vaccinia | 250 | 5,0 | > 99,99 |
| Lister | 125 | 5,0 | > 99,99 |
| | 62,5 | 3,5 | 99,93 |
| | 31,25 | 3,0 | 99,9 |
| | | | |
| Influenza | 250 | 5,0 | > 99,99 |
| A/WSN | 125 | 5,0 | > 99,99 |
| | 62,5 | 2,0 | 99,0 |

Die Erfindung wird durch die nachfolgenden Beispiele näher erläutert.

### Beispiel 1

### 3-(2-Mercaptoethyl)-chinazolin-2,4(1H,3H)-dion (Formel I, R¹ = H, R² = H, n = 1)

a) 3-(2-Hydroxyethyl)-2-methylthiochinazolin-4(3H)-thion (Formel X, R¹ = H, R² = H, R³ = CH₃ , n = 1)
   2,4 g (10 mmol) 3-(2-Hydroxyethyl)-chinazolin-2,4(1H,3H)-dithion (Formel VIII, R¹ = H, R² = H, n = 1) werden in methanolischer Natronlauge (20 ml 0,5 N NaOH und 7 ml CH₃OH) gelöst, mit 1,6 g Methyljodid (Formel IX, R³ = CH₃, X = I) versetzt und 30 Minuten im verschlossenen Gefäß bei Raumtemperatur intensiv mechanisch geschüttelt. Der gebildete Niederschlag wird abgetrennt und mit wenig 50 %igem Methanol gewaschen und getrocknet. Gelbe Kristalle.
   C₁₁H₁₂N₂OS₂ (252,4). F.: 117 - 118 °C (CH₃OH).
   Ausbeute: 92 %.
b) 2,3-Dihydro-5-oxo-6H-thiazolo[3,2-c]chinazolin-4-ium-chloridhydrat (Formel XI, R¹ = H, R² = H, n = 1, Y = Cl)
   505 mg der nach a) hergestellten. dc-reinen Verbindung werden in 9 ml methanolischer Salzsäure (30 g HCl-Gas/200 ml absolutem Methanol) gelöst und im verschlossenen Gefäß zunächst 24 Stunden bei Raumtemperatur und weitere 24 Stunden bei -20 °C aufbewahrt. Der gebildete Niederschlag wird mit absolutem Diethylether gewaschen und im Vakuumexsikkator (KOH/H₂SO₄) getrocknet. Es werden 340 bis 380 mg 2,3-Dihydro-5-oxo-thiazolo[3,2-c]chinazolin-4-ium-chloridhydrat erhalten. Gelbe Kristalle.
   C₁₀H₉ClN₂OS.H₂O (258,7). IR: C0-Bande bei 1740 cm⁻¹.
   MS: m/e 204 (Molekülionenpeak der zugrundeliegenden Base).
c) 3-(2-Mercaptoethyl)-chinazolin-2,4(1H,3H)-dion (Formel I, R¹ = H, R² = H, n = 1)
   700 mg der nach b) erhaltenen Verbindung werden in ethanolischer Natronlauge (5 ml 3 N NaOH, 35 ml Wasser und 20 ml Ethanol) unter Rühren gelöst. Nach etwa 15 Minuten wird die Reaktionslösung filtriert und zu dem farblosen Filtrat unter Rühren bis zur Beendigung der Niederschlagsbildung verdünnte Salzsäure zugefügt. Der gebildete Niederschlag wird gründlich mit Wasser gewaschen und im Vakuumexsikkator (konzentrierte H₂SO₄) getrocknet. Es werden 610 mg dc-reines (Fließmittel: Toluol/Aceton/Methanol 7 : 3 : 1; v/v/v)) Endprodukt erhalten. Farblose Kristalle.
   C₁₀H₁₀N₂O₂S (222,2). F.: 192 - 193 °C (CHCl₃/Petrolether).
   Ein weiterer Anteil an Endprodukt, mit geringen Teilen Bis [2-(2,4-dioxo-1,2,3,4-tetrahydrochinazolin-3-yl)-ethyl]-disulfan (Formel XV, R¹ = H, R² = H, n = 1) verunreinigt, kann dadurch gewonnen werden, daß das nach Abtrennung des kristallinen Thiazolochinazoliniumchloridhydrates erhaltene Filtrat bis zur Trockne eingeengt und der Rückstand in analoger Weise wie unter c) beschrieben behandelt wird.

### Beispiel 2

### 3-(3-Mercaptoprop-1-yl)-6,7-dimethoxychinazolin-2,4(1H,3H)-dion (Formel I, R¹ = 6,7-Dimethoxy, R² = H, n = 2)

a) 6,7-Dimethoxy-2-thioxo-3,1-benzoxazin-4(1H)-on
   12,5 g (0,15 ml) Thiophosgen werden als 10 %ige Dioxanlösung zur Mischung aus 19,9 g (0,1 mol) 2-Amino-4,5-dimethoxybenzoesäure, 200 ml Dioxan und 32 g (0,3 mol) Triethylamin unter Eiskühlen und Rühren zugetropft. Das Rühren wird 3 Stunden bei Raumtemperatur fortgesetzt. Anschließend wird der Reaktionsansatz mit 1000 ml Wasser versetzt und mit verdünnter Salzsäure angesäuert. Der gebildete Niederschlag wird aus Dioxan/Wasser umkristallisiert. Gelbe Kristalle.
   C₁₀H₉NO₄S (239,2). F.: 211 - 213 °C (Dioxan/Wasser).
   Ausbeute: 75 %.
b) 6,7-Dimethoxy-3,1-benzothiazin-2,4(1H)-dithion (Formel VII, R¹ = 6,7-Dimethoxy)
   2,0 g des nach a) erhaltenen 3,1-Benzoxazin-Derivates werden in der gerade ausreichenden Menge Pseudocumol heiß gelöst, mit 2,0 g Phosphor(V)-sulfid versetzt und 20 Minuten am Rückfluß erhitzt. Nach Zugabe von weiteren 1,0 g Phosphor(V)-sulfid wird weitere 15 Minuten am Rückfluß erhitzt. Die noch heiße Reaktionslösung wird filtriert. Nach 12-stündigem Stehen wird der Niederschlag abgesaugt, mit wenig Methanol gewaschen und in 0,5 N Natronlauge gelöst. Danach wird ohne Verzug die alkalische Lösung in überschüssige verdünnte Salzsäure filtriert und der gebildete Niederschlag nach Abtrennung mit Wasser gewaschen.
   Rote Kristalle. C₁₀H₉NO₂S₃ (271,4). F.: 247 - 249 °C (Ethylacetat). Ausbeute: 60 %.
c) 3-(3-Hydroxyprop-1-yl)-6,7-dimethoxychinazolin-2,4(1H,3H)-dithion (Formel VIII, R¹ = 6,7-Dimethoxy, R² = H, n = 2)
   2,7 g (10 mmol) des nach b) erhaltenen 3,1-Benzothiazin-Derivates werden zusammen mit 1,2 g Triethylamin, 0,82 g (11 mmol) 3-Aminopropan-1-ol (Formel IV, R² = H, n = 2) und 12 ml Ethanol 2 Stunden bei 60 °C am Rückfluß erhitzt. Nach dem Erkalten wird mit verdünnter Salzsäure angesäuert, der Niederschlag nach 12 Stunden abgesaugt und mit Wasser gewaschen. Gelborangefarbene Kristalle.
   C₁₃H₁₆N₂O₃S₂ (312,4). F.: 239 °C (Propan-2-ol).
   Ausbeute: 61 %.
   Die weitere Umsetzung des nach c) erhaltenen Dithions zum Endprodukt erfolgt in analoger Weise wie bei Beispiel 1 unter a) bis c) aufgeführt:
d) 3-(3-Hydroxyprop -1-yl)-6,7-dimethoxy-2-methylthiochinazolin-4(3H)-thion (Formel X, R¹ = 6,7-Dimethoxy, R² = H, R³ = CH₃, n = 2)
   Gelbe Kristalle. C₁₄H₁₈N₂O₃S₂ (326,4). F.: 168 - 170 °C.
   Ausbeute: 76 %.
e) 3,4-Dihydro-9,10-dimethoxy-6-oxo-2H,7H-1,3-thiazino-[3,2-c]chinazolin-5-iumchlorid (Formel XI, R¹ = 9,10-dimethoxy R² = H, n = 2, Y = Cl)
   Gelbliche Kristalle. C₁₃H₁₅ClN₂O₃S (314,8). IR: CO-Bande bei 1720 cm⁻¹. F.: 197 - 198 °C (nach Waschen mit Diethylether). Ausbeute: 74 %.
f) 3-(Mercapto prop-1-yl)-6,7-dimethoxy-chinazolin-2,4(1H,3H)-dion (Formel 1, R¹ = 6,7-Dimethoxy, R² = H, n = 2)
   Farblose Kristalle. C₁₃H₁₆N₂O₄S (296,3). F.: 236 °C (Methylglycol). IR: SH-Bande bei 2540 cm⁻¹, Ausbeute: 81 %

### Beispiel 3

### 3-(2-Mercaptoethyl)-6,7-dimethoxychinazolin-2,4(1H,3H)-dion (Formel 1, R¹ = 6,7-Dimethoxy, R² = H, n = 1)

Farblose Kristalle. C₁₂H₁₄N₂O₄S (282,4). IR: SH-Bande bei 2520 cm⁻¹; CO-Banden bei 1697 und 1645 cm⁻¹. F.: 312 - 314 °C (Chloroform/Petrolether). Ausbeute: 84 % (bezogen auf den letzten Syntheseschritt).

Die Darstellung dieser Verbindung erfolgte aus 6,7-Dimethoxy-3,1-benzothiazin-2,4(1H)-dithion und 2-Aminoethan -1-ol analog den Beispielen 1 und 2. Dabei wurden folgende Zwischenstoffe isoliert und charakterisiert:
a) 3-(2-Hydroxyethyl)-6,7-dimethoxychinazolin-2,4-(1H,3H)-dithion (Formel VIII, R¹ = 6,7-Dimethoxy, R² = H, n = 1)
   Gelborangefarbene Kristalle. C₁₂H₁₄N₂O₃S₂ (298,4). F.: 181 - 183 °C (Dioxan/Wasser). Ausbeute: 92 %.
b) 3-(2-Hydroxyethyl)-6,7-dimethoxy-2-methylthiochinazolin-4(3H)-thion (Formel X, R¹ = 6,7-Dimethoxy, R² = H, R³ = CH₃, n = 1)
   Gelbe Kristalle. C₁₃H₁₆N₂O₃S₂ (312,4). F.: 187 - 188 °C (Propan-2-ol). Ausbeute 72 %.
c) 8,9-Dimethoxy-5-oxo-2,3-dihydro-6H-thiazolo[3,2-c]chinazolin-4-ium-chlorid (Formel XI, R¹ = 8,9-Dimethoxy, R² = H, n = 1, Y = Cl).
   Gelbliche Kristalle. C₁₂H₁₃ClN₂O₃S (300,8). IR: CO-Bande bei 1715 cm⁻¹. F.: 201 - 205 °C (nach Waschen mit Diethylether). Ausbeute: 67 %.

### Beispiel 4

### 3-(2-Mercaptoethyl)-chinazolin-2,4(1H,3H)-dion (Formel I, R¹ = H, R² = H, n = 1)

a) Bis [2-(2-amino benzoylamino)-ethyl]-disulfan (Formel XIII, R¹ = H, R² = H, n = 1)
   6,5 g (40 mmol) 2H-3,1-Benzoxazin-2,4(1H)-dion (Formel III, R¹ = H) werden in 30 ml Dimethylformamid und mit der aus 4,5 g (20 mmol) Cystaminiumchlorid (Cystamindihydrochlorid, (Formel XII, R² = H, n = 1, . 2 HCl), 20 ml Dimethylformamid und 6 g (60 mmol) Triethylamin bereiteten Suspension versetzt. Der Ansatz wird 45 Minuten im Wasserbad auf 70 °C erwärmt. Nach dem Erkalten wird der Niederschlag abgetrennt und mit 20 ml Dimethylformamid gewaschen. Das Filtrat und die Waschlösung werden vereinigt und in 150 ml Wasser gegossen. Nach mehrstündiger Aufbewahrung im Kühlschrank wird der Niederschlag abgesaugt, mit Wasser gewaschen und getrocknet. Es werden 7,0 g (90 % der Theorie) dc-reines Bis [2-(2-aminobenzoylamino)-ethyl]-disulfan erhalten, das ohne weitere Reinigung für die nachfolgende Reaktion verwendet werden kann. F.: 129 - 131 °C (Ethanol/Wasser).
b) Bis [2-(2,4-dioxo-1,2,3,4-tetrahydrochinazolin-3-yl)-ethyl]disulfan (Formel XV, R¹ = H, R² = H, n = 1)
   0,98 g (2,5 mmol) Bis [2-(2-aminobenzoylamino)-ethyl]-disulfan werden in 10 ml Pyridin gelöst. Unter Eiskühlung wird 1 ml (10 mmol) Chlorameisensäureethylester (Formel XIV R⁴ = C₂H₅) zugetropft. Der Ansatz wird 1 Stunde am Rückfluß erhitzt und nach dem Erkalten zu der aus 15 ml HCl (1 mol/l) und 10 ml Eiswasser bereiteten Mischung gegeben. Nach längerer Aufbewahrung im Kühlschrank wird der Niederschlag abgesaugt, mit Wasser gewaschen und getrocknet. Das erhaltene Zwischenprodukt wird in gepulverter Form in einer Mischung aus 8 ml NaOH (3 mol/l) und 1,6 ml Ethanol suspendiert, 6 Stunden in einem verschlossenen Reaktionsgefäß geschüttelt und weitere 24 Stunden bei Raumtemperatur aufbewahrt. Es werden 30 ml warmes Wasser zugegeben und der Ansatz filtriert. Das Filtrat wird mit verdünnter HCl angesäuert, der Niederschlag wird abgesaugt und getrocknet.
   Es werden 0,85 g (77 % der Theorie) dc-reines Bis [2-(2,4-dioxo-1,2,3,4-tetrahydrochinazolin-3-yl)-ethyl]-disulfan erhalten, das ohne weitere Reinigung für die nachfolgende Reaktion verwendet werden kann. F.: 270 - 272 °C (Propan-1-ol).
c) 3-(2-Mercaptoethyl)-chinazolin-2.4(1H,3H)-dion (Formel I, R¹ = H, R² = H, n = 1)
   200 mg des nach b) erhaltenen Produktes 200 mg Zinkstaub und 12 ml Essigsäure werden 3 Stunden am Rückfluß erhitzt. Der Ansatz wird mit einer aus 16 ml HCl (1 mol/l) und 28 ml Wasser bereiteten Mischung versetzt und erneut zum Sieden gebracht. Es wird filtriert und das Filtrat 1 bis 2 Tage in einem verschlossenen Gefäß im Kühlschrank aufbewahrt. Der Niederschlag wird abgesaugt und getrocknet.
   Ausbeute: 150 mg (75 % der Theorie) dc-reines Produkt.
   F.: 193 - 195 °C.

### Beispiel 5

### 6-Brom-3-(2-mercaptoethyl)-chinazolin-2,4(1H,3H)-dion (Formel I, R¹ = 6-Br, R² = H, n = 1)

F.: 298 - 300 °C, Ausbeute: 52 % (bezogen auf den letzten Syntheseschritt).

Die Darstellung dieser Verbindung erfolgte analog dem Beispiel 4. Dabei wurden folgende Zwischenstoffe isoliert und charakterisiert:
a) Bis [2-(2-amino-5-brombenzoylamino)-ethyl]-disulfan Formel XIII, R¹ = 5-Br, R² = H, n = 1)
   Ausbeute: 47 % (nach Umkristallisation aus Ethanol).
   F.: 190 -195 °C (Chloroform/n-Hexan (1 : 1)).
b) Bis[2-(6-brom-2,4-dioxo-1,2,3,4-tetrahydro-chinazolin-3-yl)-ethyl]-disulfan (Formel XV, R¹ = 6-Br, R² = H, n = 1)
   Ausbeute 60 % (nach Umkristallisaiton aus Dimethylformamid).
   F.: 334 - 336 °C (Dimethylformamid).

### Beispiel 6

### 3-(3-Mercaptopropyl)-chinazolin-2,4(1H,3H)-dion (Formel I, R¹ = H, R² = H, n = 2)

24,5 g 2H-3,1-Benzoxazin-2,4(1H)-dion (Formel III, R¹ = H) werden innerhalb von 20 Minuten in eine Lösung aus 12,3 g 3-Aminopropan-1-ol (Formel IV, R² = H, n = 2) und 45 ml Wasser unter Rühren eingetragen und anschließend 10 Minuten auf dem siedenden Wasserbad erwärmt (= Lösung 1).

17,0 g Kaliumhydroxid werden in 225 ml Ethanol unter Erwärmen gelöst. Zu der erkalteten Lösung werden 36 ml Schwefelkohlenstoff hinzugefügt. Danach wird der Ansatz 15 Minuten im verschlossenen Gefäß stehen gelassen, mit der Lösung 1 vereinigt und anschließend unter einem Abzug 3,5 Stunden am Rückfluß erhitzt. Danach werden ca. 70 - 80 ml des Lösungsmittelgemisches abdestilliert, der Reaktionsansatz nach dem Erkalten mit Eisessig angesäuert und mindestens 3 Stunden zur Kristallisation stehen gelassen. Es werden 24,3 g dc-reines 3-(3-Hydroxypropyl)-2-thioxo-2,3-dihydro-chinazolin-4(1H)-on (Formel VI, R¹ = H. R² = H, n = 2) erhalten. F.: 174 - 176 °C.

Nach Zugabe von ca. 200 ml Wasser zur Mutterlauge können weitere 4,0 g nahezu dc-reines Rohprodukt gewonnen werden. Eine Umkristallisation ist aus Ethanol möglich.

30,0 g des vorstehend erhaltenen trockenen Rohproduktes werden unter einem Abzug zusammen mit 300 ml konzentrierter Salzsäure 2,5 Stunden am Rückfluß erhitzt. Dabei ist für eine Entsorgung der entweichenden HCl-Dämpfe zu sorgen. Anschließend werden zu dem Reaktionsansatz 2,7 l Wasser hinzugefügt, der weitere 20 Stunden am Rückfluß gehalten wird. Nach dem Erkalten wird der Niederschlag abgesaugt und an der Luft oder bei ca. 30 - 40 °C im Trockenschrank getrocknet. Die Umkristallisation erfolgt aus Propan-1-ol. Ausbeute Reinprodukt: 83 %. F.: 165 - 167 °C.

In einer zweiten Verfahrensvariante können 30,0 g 3-(3-Hydroxypropyl)-2-thioxo-2,3-dihydrochinazolin-4(1H)-on (Formel VI, R¹ = H, R² = H, n = 2) mit einer Mischung aus 30 ml Eisessig und 12 ml konzentrierter Schwefelsäure erhitzt werden. Nach Zugabe von ca. 330 ml Wasser wird das Erhitzen unter Rückfluß 15 Stunden fortgesetzt. Die Aufarbeitung und Reinigung erfolgt wie bei obiger Variante. Die Ausbeute an nahezu dc-reinem Rohprodukt beträgt 85 %. F.: 165 - 167 °C (Propan-1-ol).

### Beispiel 7

### 3-(2-Mercaptopropyl)-chinazolin-2,4(1H,3H)-dion (Formel I, R¹ = H, R² = CH₃, n = 1)

24,5 g 2H-3,1-Benzoxazin-2,4(1H)-dion (Formel III, R¹ = H) werden analog Beispiel 6 mit 12,3 g 2-Aminopropan -1-ol (Formel IV, R² = CH₃, n = 1) zur Lösung 2 umgesetzt.

Unter Verwendung von 17,0 g Kaliumhydroxid, 225 ml Ethanol und 36 ml Schwefelkohlenstoff (es kann auch die entsprechende Menge Kaliummethylxanthogenat zum Einsatz gelangen) wird entsprechend Beispiel 6 verfahren, mit der Lösung 2 vereinigt und erneut analog Beispiel 6 zum nahezu dc-reinen 3-(2-Hydroxypropyl)-2-thioxo-2,3-dihydro-chinazolin-4(1H)-on (Formel VI, R¹ = H, R² = CH₃, n = 1) umgesetzt. Eine Umkristallisation ist aus Methanol möglich.

30,0 g des vorstehend erhaltenen trockenen Rohproduktes werden in völlig analoger Weise wie im Beispiel 6 beschrieben zu dem 3-(2-Mercaptopropyl)-chinazolin-2,4(1H,3H)-dion (Formel I, R¹ = H, R² = CH₃, n = 1) umgesetzt. Erhitzungsdauer mit konzentrierter Salzsäure 2 Stunden, nach Wasserzugabe nochmals 15 Stunden. Nach Umkristallisation des trockenen Rohproduktes aus Toluol werden 22,4 g Reinprodukt erhalten.
Ausbeute: 69 %. F.: 206 - 208 °C.

### Beispiel 8

### 6-Chlor-3-(3-mercaptopropyl)-chinazolin-2,4(1H,3H)-dion (Formel I, R¹ = 6-Cl), R² = H, n = 2)

Die Darstellung erfolgt analog Beispiel 6 aus 12,5 g 6-Chlor-2H-3,1-benzoxazin-2,4(1H)-dion, 5,8 g 3-Aminopropanol, 40 ml Wasser sowie 7,0 g Kaliumhydroxid, 100 ml Ethanol und 7,5 ml Schwefelkohlenstoff. Reaktionszeit 4 - 4,5 Stunden. Es werden 35 - 40 ml des Lösungsmittelgemisches abdestilliert und die Reaktionslösung filtriert. Das Filtrat wird mit Essigsäure angesäuert. Es werden 6 g rohes 6-Chlor-3-(3-hydroxypropyl)-2-thioxo-2,3-dihydro chinazolin-4(1H)-on (Formel VI, R¹ = 6-Cl, R² = H, n = 2) erhalten. Nach Zugabe von ca. 150 g Eis zum Filtrat werden weitere 1,1 g Rohprodukt gewonnen. Eine Umkristallisation des Rohproduktes kann aus Propan-1-ol erfolgen. F.: 255 - 257 °C.

3,0 g des vorstehend hergestellten Reinproduktes werden analog Beispiel 6 zum rohen 6-Chlor-3-(3-mercaptopropyl)-chinazolin-2,4(1H,3H)-dion (Formel I, R¹ = 6-Cl, R² = H, n = 2) umgesetzt. Nach Umkristallisation aus Propan-1-ol werden 1,96 g Reinprodukt mit einer Ausbeute von 66 % erhalten.

### Beispiel 9

### 3-(2-Mercaptoethyl)-chinazolin-2,4(1H,3H)-dion (Formel I, R¹ = H, R² = H, n = 1)

a) S-[2-(2,4-Dioxo-1,2,3,4-tetrahydrochinazolin-3-yl)-ethyl]-isothiuroniumchlorid (Formel XVII, R¹ = H, R² = H, n = 1, Hal = Cl)
   4,5 g (20 mmol) 3-(2-Chlorethyl)-2,4-dioxo-1,2,3,4-tetrahydrochinazoiin (Formel XVI, R¹ = H, R² = H, n = 1, Hal = Cl) werden mit 3,1 g (40 mmol) Thioharnstoff (gepulvert) in 100 ml Methylglykol 45 Minuten am Rückfluß erhitzt. Der nach 12 stündigem Stehen des Reaktionsansatzes bei 4 °C isolierte Niederschlag wird mit wenig Eiswasser gewaschen und an der Luft getrocknet.
   Es werden 5,1 g dc-reines S-[2-(2,4-Dioxo-1,2,3,4-tetrahydrochinazolin-3-yl)-ethyl]-isothiuroniumchlorid erhalten. Ausbeute: 85 %. F.: 259 - 262 °C (nach Auskochen mit absolutem Ethanol). Fließmittelsystem für die Dünnschichtchromatographie: Toluol/Aceton/Methanol 7 : 2 : 1.
b) 3-(2-Mercaptoethyl)-chinazolin-2,4(1H,3H)-dion
   2,84 g (10 mmol) der nach a) erhaltenen Verbindung werden unter N₂-Begasung in 150 ml Natronlauge (0,25 mol/l) bei einer Temperatur von -5 bis +5 °C eingetragen und bis zur vollständigen Umsetzung (maximal 20 Minuten) unter Fortsetzung der N₂-Gas-Einleitung gerührt (dc-Kontrolle; Ansatz wird zwischenzeitlich bei -18 °C aufbewahrt). Anschließend wird die Reaktionslösung filtriert, mit ca. 45 ml Salzsäure angesäuert und etwa 6 - 10 Stunden bei ca. 4 °C in einem verschlossenem Gefäß aufbewahrt. Der abgetrennte Niederschlag wird mit Wasser gewaschen und nach dem Trocknen an der Luft aus Propan-1-ol bzw. Eisessig umkristallisiert.
   F.: 192 - 194 °C (Propan-1-ol).

### Beispiel 10

### 3-(3-Mercaptopropyl)-chinazolin-2,4(1H,3H)-dion (Formel I, R¹ = H, R² = H, n = 2)

a) S-[3-(2,4-Dioxo-1,2,3,4-tetrahydro-chinazolin-3-yl)-propyl]-isothiuroniumchlorid (Formel XVII, R¹ O H, R² = H, n = 2, Hal = Cl)
   0,48 g (2 mmol) 3-(3-Chlorpropyl)-2,4-dioxo-1,2,3,4-tetrahydrochinazolin (Formel XVI R¹ = H, R² = H, n = 2, Hal = Cl) werden mit 0,31 g (4 mmol) gepulvertem Thioharnstoff 3 Stunden am Rückfluß erhitzt. Der nach Zugabe von absolutem Diethylether gebildete Niederschlag wird mit wenig Eiswasser gewaschen. Nach dem Trocknen an der Luft werden 0,29 g reines S-[3-(2,4-Dioxo-1,2,3,4-tetrahydrochinazolin-3-yl)-propyl]-isothiuroniumchlorid erhalten.
   Ausbeute: 46 %. F.: 224 - 228 °C (nach Auskochen mit absolutem Ethanol).
b) 3-(3-Mercaptopropyl)-chinazolin-2,4(1H,3H)-dion
   Diese Verbindung wird aus der nach a) dieses Beispiels erhaltenen Verbindung durch analoge Umsetzung wie in Beispiel 9b) beschrieben erhalten.
   F.: 165 - 167 °C (Propan-1-ol).

### Beispiel 11

### 3-(2-Mercaptopropyl)-chinazolin-2,4(1H,3H)-dion (Formel I, R¹ = H, R² = CH₃, n =1)

a) S-[2-(2,4-Dioxo-1,2,3,4-tetrahydrachinazolin-3-yl)-propyl]-isothiuronium chlorid (Formel XVII, R¹ = H, R² = CH₃, n = 1, Hal = Cl)
   Die Darstellung erfolgt aus 3-(2-Chlorpropyl)-2,4-dioxo-1,2,3,4-tetrahydrochinazolin (Formel XVI, R¹ = H, R² = CH₃, n = 1, Hal = Cl) analog Beispiel 10a) durch 75minutiges Erhitzen am Rückfluß.
   Ausbeute: 0,32 g (51 % der Theorie). F.: 225 - 232 °C (nach Auskochen mit absolutem Ethanol).
b) 3-(2-Mercaptopropyl)-chinazolin-2,4(1H,3H)-dion
   Diese Verbindung wird aus der nach a) dieses Beispiels erhaltenen Verbindung durch analoge Umsetzung wie in Beispiel 9b) beschrieben erhalten.
   F.: 206 - 208 °C (Toluol).

### Beispiel 12

### 6-Methyl-3-(3-mercaptopropyl)-chinazolin-2,4(1H,3H)-dion (Formel I, R¹ = 6-CH₃, R² = H, n = 2)

a) S-[3-(6-Methyl-2,4-dioxo-1,2,3,4-tetrahydrochinazolin-3-yl)-propyl]-isothiuroniumchlorid (Formel XVII, R' = CH₃, R² = H, n = 2, Hal = Cl)
   Die Darstellung erfolgte analog Beispiel 10a) aus 0,506 g (2 mmol) 6-Methyl-3-(3-chlorpropyl)-2,4-dioxo-1,2,3,4-tetrahydrochinazolin (Formel XVI, R¹ = CH₃, R² = H, n = 2, Hal = Cl), 0,17 g (2,2 mmol) gepulvertem Thioharnstoff und 4 ml Methylglykol. Nach 12 stündigem Aufbewahren bei -15 °C werden 0,25 g und nach Zugabe von absolutem Diethylether zum Filtrat nochmals 0,08 g S-[3-(6-Methyl-2,4-dioxo-1,2,3,4-tetrahydrochinazolin 3-yl)-propyl]-isothiuroniuchlorid erhalten.
   Ausbeute: 49 %. F.: 151 - 153 °C (nach Auskochen mit absolutem Ethanol).
   Das als Ausgangsprodukt eingesetzte 6-Methyl-3-(3-chlorpropyl)-2,4-dioxo-1,2,3,4-tetrahydrochinazolin wurde durch Umsetzung von 6-Methyl-3-(3-hydroxypropyl)-2,4-dioxo-1,2,3,4-tetrahydrochinazolin (F.: 196 - 197 °C (Ethanol, Kristallumwandlung bei 182 -184 °C)). mit Thionylchlorid nach Grout und Partridge (J. Chem. Soc. (London) 1960, 3546) dargestellt. F.: 194 - 196 °C (Ethanol). Ausbeute: 91 %.
b) 6-Methyl-3-(3-mercaptopropyl)-chinazolin-2,4(1H,3H)-dion
   Diese Verbindung wird aus der nach a) dieses Beispiels erhaltenen Verbindung durch analoge Umsetzung wie in Beispiel 9b) beschrieben erhalten.
   F.: 272 - 274 °C (Propan-1-ol).

### Beispiel 13

### 6-Chlor-3-(2-mercaptoethyl)-chinazolin-2,4(1H,3H)-dion (Formel I, R¹ = 6-Cl, R² = H, n = 1)

Die Herstellung dieser Verbindung erfolgt in analoger Weise wie in den Beispielen 9 bis 12 beschrieben.
F.: 331 - 333 °C (Eisessig).

### Beispiel 14

Analog den Beispielen 1 bis 13 wurden folgende Verbindungen hergestellt:
a) 6-Methyl-3-(2-mercaptoethyl)-chinazolin-2,4(1H,3H)-dion,
   F.: 309 - 311 °C (Propan-1-ol)
b) 6-Brom-3-(3-mercaptopropyl)-chinazolin-2 4(1H 3H)-dion.
   F.: 220 - 221 °C (Eisessig)
c) 6-Fluor-3-(2-mercaptoethyl)-chinazolin-2,4(1H 3H)-dion.
   F.: 240 - 242 °C (Propan-1-ol)
d) 6-Fluor-3-(3-mercaptopropyl)-chinazolin-2,4(1H,3H)-dion.
   F.: 180 - 181 °C (Propan-1-ol)
e) 6,7-Dimethoxy-3-(2-mercaptopropyl)-chinazolin-2,4(1H,3H)-dion.
   F.: 262 - 264 °C (Ethanol)

### Beispiel 15

Tabletten mit 45,0 mg 3-(2-Mercaptoethyl)-chinazolin-2,4(1H,3)-dion

### Zusammensetzung:

1 Tablette enthält 45,0 mg Wirkstoff, 35.0 mg Lactose, 25.0 mg Kartoffelstärke, 3,5 mg Polyvinylpyrrolidon und 1,5 mg Magnesiumstearat.

### Herstellungsverfahren:

Der mit Kartoffelstärke und Lactose vermischte gepulverte Wirkstoff wird mit einer 20 %igen ethanolischen Lösung des Polyvinylpyrrolidons gleichmäßig durchfeuchtet, durch ein Sieb der Maschenweite 1,5 mm gedrückt, bei 40 °C getrocknet und erneut durch ein Sieb (Maschenweite 1,0 mm) gepreßt. Das erhaltene Granulat wird mit Magnesiumstearat gemischt und zu Tabletten verpreßt.
Tabletten masse: 110 mg.

### Beispiel 16

Dragees mit 30,0 mg 3-(2-Mercaptoethyl)-chinazolin-2,4(1H,3H)-dion

### Zusammensetzung:

1 Drageekern enthält 30,0 mg Wirkstoff, 30,0 mg Lactose, 16,5 mg Maisstärke, 2,8 mg Polyvinylpyrrolidon und 0,7 mg Magnesiumstearat.

### Herstellungsverfahren:

Die mit Lactose und Maisstärke vermischte pulverförmige Wirksubstanz wird mit einer 20 %igen ethanolischen Lösung des Polyvinylpyrrolidons gleichmäßig durchfeuchtet, durch ein Sieb der Maschenweite 1,5 mm gedrückt. bei 40 °C getrocknet und nochmals durch ein Sieb (Maschenweite 1.0 mm) gepreßt. Das erhaltene Granulat wird mit Magnesiumstearat gemischt und auf übliche Weise zu Drageekernen verpreßt.
Kernmasse: 80,0 mg

Die hergestellten Drageekerne werden nach üblichen Verfahren mit einer Hülle überzogen und auf übliche Weise poliert.

## Patentansprüche

1. 3-(Mercaptoalkyl)-chinazolin-2,4(1H,3H)-dione (1H,3H)-dione der allgemeinen Formel I, 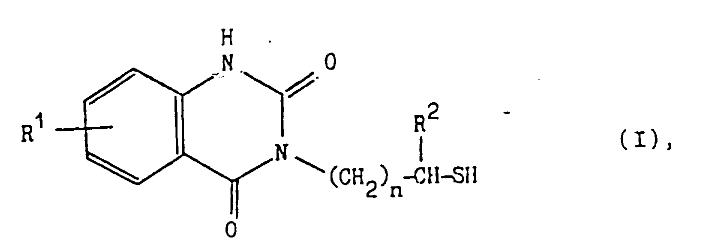 worin bedeuten:
R¹ Wasserstoff, 6-Methyl, 6-Fluor, 6-Chlor, 6-Brom oder 6,7-Dimethoxy,
R² Wasserstoff oder Methyl
und
n 1 oder 2,
einschließlich ihrer pharmazeutisch geeigneten Salze sowie ihre Tautomeren.

2. 3-(Mercaptoalkyl)-chinazolin-2,4(1H,3H)-dione nach Anspruch 1 der allgemeinen Formel II 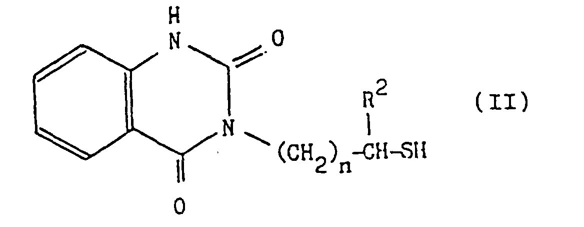 mit R² und n wie in Anspruch 1
einschließlich ihrer pharmazeutisch geeigneten Salze sowie ihre Tautomeren.

3. Verfahren zur Herstellung der Verbindungen der allgemeinen Formeln I und II nach den Ansprüchen 1 und 2,
gekennzeichnet durch folgende Verfahrensweisen:
(A) Umsetzung von 2H-3,1-Benzoxazin-2,4(1H)-dionen der allgemeinen Formel III, 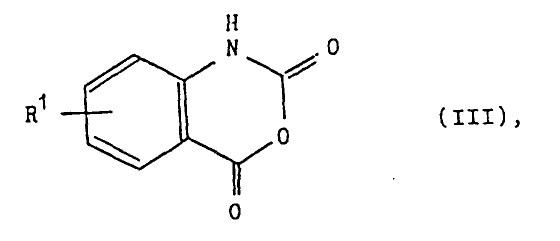 worin R¹ dasselbe wie in Anspruch 1 bedeutet,
mit einem Aminoalkanol der allgemeinen Formel IV, 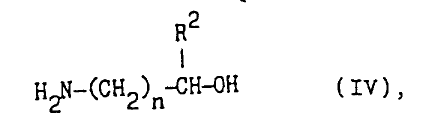 worin R² und n dasselbe wie in Anspruch 1 bedeuten,
zu einer Verbindung der allgemeinen Formel V, 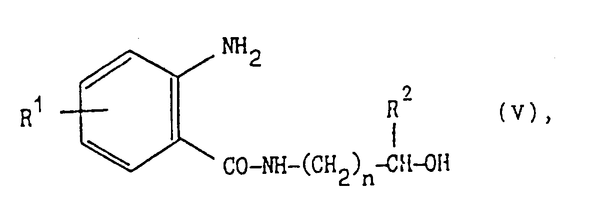 worin R¹, R² und n dasselbe wie oben bedeuten,
- Zugabe einer Reaktionslösung aus einem C₁₋₃-Alkanol, Schwefelkohlenstoff und Natrium- oder Kaliumhydroxid bzw. Natrium- oder Kaliumxanthogenat,
- Erhitzen des Reaktionsgemisches,
- Abkühlen und Ansäuern des Reaktionsgemisches,
- Erhitzen der erhaltenen Verbindungen der allgemeinen Formel VI, 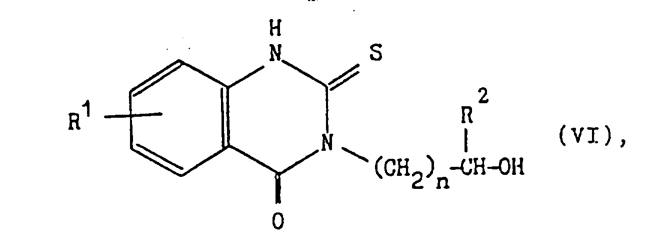 worin R¹, R² und n dasselbe wie oben bedeuten, mit konzentrierter Mineralsäure, wie Salzsäure und/oder Schwefelsäure, oder Mischungen dieser Mineralsäuren mit Eisessig und/oder Ameisensäure,
- und ggfs. anschließende Zugabe von Wasser zum Reaktionsansatz und erneutes Erhitzen unter Rückfluß unter Erhalt der Verbindungen der allgemeinen Formel I oder ihrer Tautomeren
oder
(B)
- Erhitzen von 4H-3,1-Benzothiazin-2,4(1H)-dithionen der allgemeinen Formel VII, 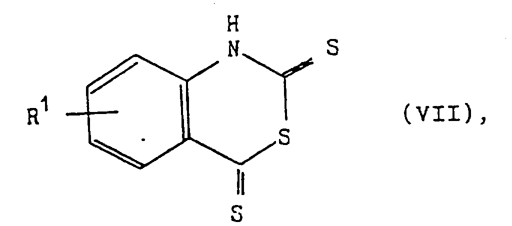 worin R¹ dasselbe wie oben bedeutet,
mit einem Aminoalkanol der allgemeinen Formel IV, 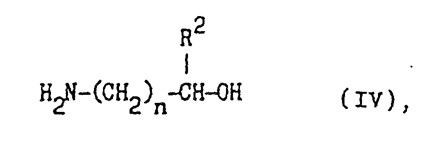 worin R² und n dasselbe wie oben bedeuten,
in einem polaren organischen Lösungsmittel unter Erhalt der Verbindungen der allgemeinen Formel VIII, 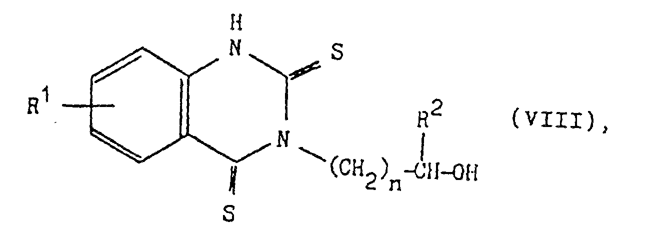 worin R¹, R² und n dasselbe wie oben bedeuten,
- Umsetzung der Verbindungen der Formel VIII in einem wäßrig-alkanolischen Reaktionsmilieu in Gegenwart eines Säureakzeptors, wie eines Alkalihydroxids oder Triethylamin, mit einem Alkylhalogenid der allgemeinen Formel IX,
R³-X (IX)
worin
R³ C₁₋₃-Alkyl
und
X Jod oder Brom
bedeuten,
zu den Verbindungen der allgemeinen Formel X, 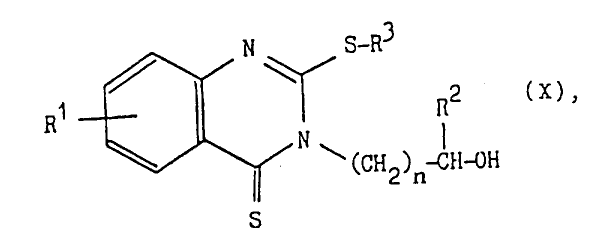 worin R¹, R², R³ und n dasselbe wie oben bedeuten,
- Umsetzung der Verbindungen der Formel X mit alkanolischer Mineralsäure zu tricyclischen Chinazoliniumsalzen der allgemeinen Formel XI, 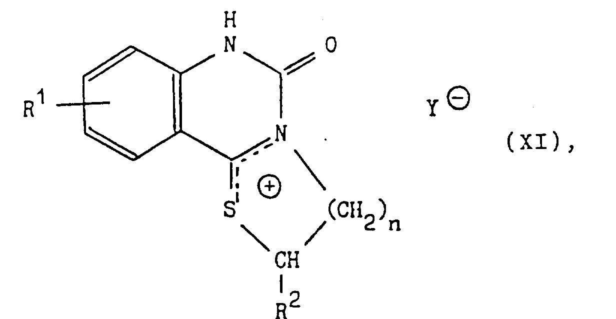 worin
R¹, R² und n dasselbe wie oben bedeuten
und
Y ein Säureanion, vorzugsweise Chlor, darstellt,
und
- Überführung der Verbindungen der Formel XI durch Behandlung in alkanolisch-wäßriger Natronlauge, nachfolgende Filtration und Ansäuern des Filtrats mit verdünnter Mineralsäure in die Verbindungen der allgemeinen Formel I oder ihre Tautomeren
oder
(C)
- Umsetzung von 2H-Benzoxazin-2,4(1H)-dionen der allgemeinen Formel III, 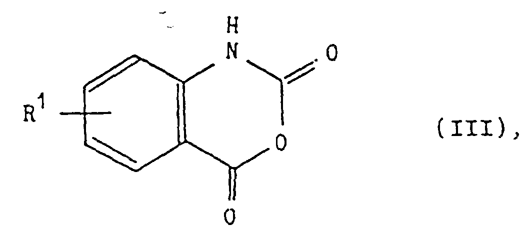 worin R¹ dasselbe wie oben bedeutet,
mit Bis(aminoalkyl)-disulfanen der allgemeinen Formel XII, 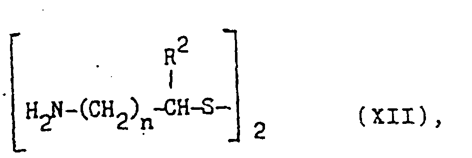 worin R² und n dasselbe wie oben bedeuten,
oder deren Salzen in einem Lösungsmittel zu Verbindungen der allgemeinen Formel XIII, 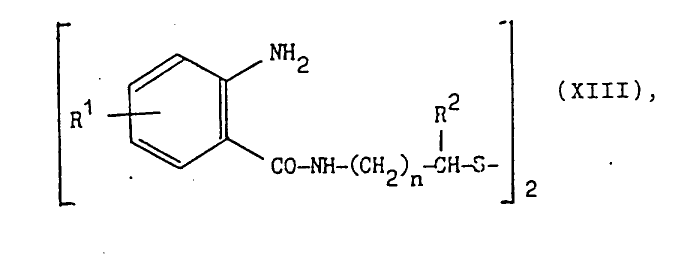 worin R¹, R² und n dasselbe wie oben bedeuten,
- Umsetzung der Verbindungen der Formel XIII mit Chlorameisensäurealkylestern der allgemeinen Formel XIV,
C1-COOR⁴ (XIV),
worin R⁴ C₁₋₃-Alkyl bedeutet,
in einem Lösungsmittel oder Lösungsmittelgemisch zu Verbindungen der allgemeinen Formel XV, 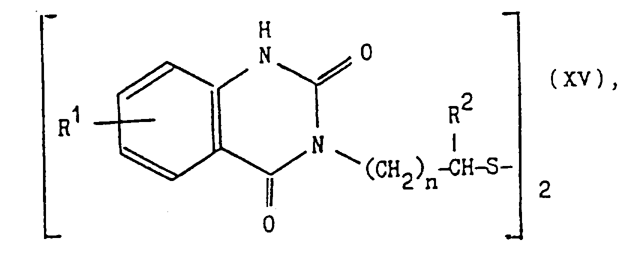 worin R¹, R² und n dasselbe wie oben bedeuten,
und
- Überführung der Verbindungen der Formel XV, gegebenenfalls nach Reinigung und Trocknung, in einem Lösungsmittel oder Lösungsmittelgemisch mit nascierendem Wasserstoff in die Verbindungen der allgemeinen Formel I oder ihre Tautomeren
oder
(D)
- Umsetzung von 3-(Haloalkyl)-2,4-dioxo-1,2,3-tetrahydrochinazolinen der allgemeinen Formel XVI, 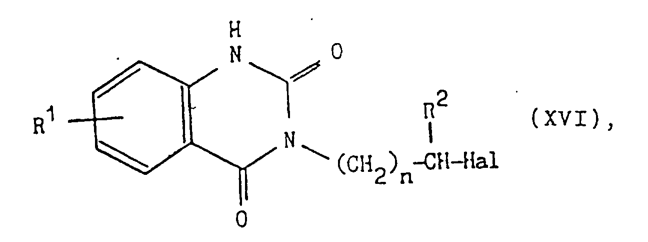 worin
R¹, R² und n dasselbe wie oben bedeuten
und
Hal Chlor oder Brom darstellt,
mit Thioharnstoff zu den S-[ω-(2,4-Dioxo-1,2,3,4-tetrahydrochinazolin-3-yl)-alkyl]-isothiuroniumhalogeniden der allgemeinen Formel XVII, 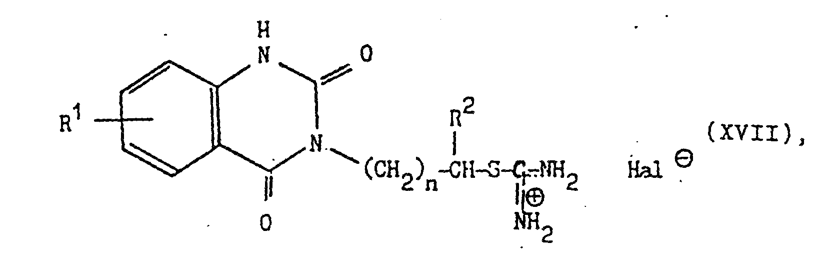 worin R¹, R², n und Hal dasselbe wie oben bedeuten,
und
- Überführung der Verbindungen der Formel XVII, gegebenenfalls nach ihrer Abtrennung, mit Alkalilauge in die Verbindungen der allgemeinen Formel I oder ihre Tautomeren
sowie gegebenenfalls Überführung der nach den Verfahrensweisen A-D erhaltenen Verbindungen der allgemeinen Formel I in entsprechende Salze, wie die Alkalisalze oder Ammoniumsalze.

4. Verfahren nach Anspruch 3, gekennzeichnet durch eine oder mehrere der nachstehenden Maßnahmen:
(a) Die Umsetzung der Verbindungen der Formel III mit den Verbindungen der Formel IV wird in einem wäßrigen Reaktionsmilieu durchgeführt;
(b) die Umsetzung der Verbindungen der Formel VIII mit den Verbindungen der Formel IX wird bei Raumtemperatur durchgeführt;
(c) die Umsetzung der Verbindungen der Formel X zu den Verbindungen der Formel XI wird mit absoluter ethanolischer Salzsäure vorgenommen;
(d) die Verbindungen der Formel III werden nach Variante (C) mit den Dihydrochloriden der Verbindungen der Formel XII umgesetzt;
(e) die Umsetzung der Verbindungen der Formel XVI mit Thioharnstoff erfolgt im Temperaturbereich von 80 bis 150 °C;
(f) die Überführung der Verbindungen der Formel XVII in die Verbindungen der Formel I bzw. deren Tautomere erfolgt in Gegenwart eines Schutzgases;
(g) die Überführung der Verbindungen der Formel XVII in die Verbindungen der Formel I bzw. deren Tautomere erfolgt im Temperaturbereich von -10 bis +15 °C.

5. Verfahren nach Anspruch 3(A) und/oder 4, dadurch gekennzeichnet, daß die aus den Umsetzungen der Verbindungen der allgemeinen Formel VI mit konzentrierten Mineralsäuren, wie Salzsäure oder Schwefelsäure, oder Mischungen dieser Mineralsäuren mit Eisessig und/oder Ameisensäure resultierenden Feststoffgemische abgetrennt und nachfolgend durch Erhitzen in verdünnten Mineralsäuren in die Verbindungen der allgemeinen Formel I oder deren Tautomere übergeführt werden.

6. Verfahren nach Anspruch 3(D) und/oder 4, dadurch gekennzeichnet, daß die Umsetzung der Verbindungen der allgemeinen Formel XVI mit Thioharnstoff in einem Molverhältnis von 1:1 bis 1:3 vorgenommen wird.

7. Verfahren nach einem oder mehreren der Ansprüche 3(D), 4 und 5, dadurch gekennzeichnet, daß zur Herstellung der Verbindungen der allgemeinen Formel XVII die Reaktionspartner in einem protischen Lösungsmittel unter Erhitzen am Rückfluß umgesetzt werden und/oder die Umsetzung reaktionsträger Verbindungen der allgemeinen Formel XVI zwischen 100 und 150 °C, gegebenenfalls im Vakuum, vorgenommen wird.

8. Verfahren nach Anspruch 7, dadurch gekennzeichnet, daß zur Herstellung der Verbindungen der allgemeinen Formel XVII die Reaktionspartner in Methylglykol oder Butan-1-ol umgesetzt werden.

9. Verfahren nach einem oder mehreren der Ansprüche 3(D) und 6 bis 8, dadurch gekennzeichnet, daß die Umsetzung der Verbindungen der allgemeinen Formel XVII zu den Verbindungen der allgemeinen Formel I mit Natronlauge einer Konzentration von 0,1 bis 1,0 mol/l, gegebenenfalls unter mechanischem Rühren oder Schütteln, vorgenommen wird.

10. Verfahren nach Anspruch 9, dadurch gekennzeichnet, daß die Umsetzung der Verbindungen der allgemeinen Formel XVII zu den Verbindungen der allgemeinen Formel I mit Natronlauge einer Konzentration von 0,25 mol/l vorgenommen wird.

11. Pharmazeutische Zubereitungen, gekennzeichnet durch eine oder mehrere Verbindungen der allgemeinen Formel I nach Anspruch 1 und/oder eine oder mehrere Verbindungen der allgemeinen Formel II nach Anspruch 2 sowie inerte, pharmazeutisch geeignete Hilfs- und/oder Trägerstoffe.

12. Pharmazeutische Zubereitungen, gekennzeichnet durch 3-(2-Mercaptoethyl)-chinazolin-2,4(1H,3H)-dion und/oder sein Tautomer und/oder pharmazeutisch geeignete Salze dieser Verbindungen als Wirkstoffe.

13. Verfahren zur Herstellung der pharmazeutischen Zubereitungen nach den Ansprüchen 11 und 12, dadurch gekennzeichnet, daß man eine oder mehrere Verbindungen der allgemeinen Formel I nach Anspruch 1 bzw. eine oder mehrere Verbindungen der allgemeinen Formel II nach Anspruch 2 mit inerten, pharmazeutisch üblichen Hilfs- und/oder Trägerstoffen in an sich bekannter Weise in eine für die medizinische Verabfolgung geeignete Darreichungsform, wie Tabletten, Dragees, Kapseln, Suppositorien, Lösungen oder Ampullen, bringt.

14. Verwendung der Verbindungen der allgemeinen Formeln I und II nach den Ansprüchen 1 bzw. 2 zur Herstellung pharmazeutischer Zubereitungen zur Behandlung von Immunerkrankungen, mit Einschluß der Erzielung einer immunstimulierenden und/oder immunrestaurativen Wirkung.

15. Verwendung der Verbindungen der allgemeinen Formeln I und II nach den Ansprüchen 1 bzw. 2 zur Herstellung pharmazeutischer Zubereitungen zur Behandlung von Virusinfekten, mit Einschluß der Erzielung einer antiviralen Wirkung, in der Human- und Veterinärmedizin.

## Claims

1. 3-(Mercaptoalkyl) quinazoline-2,4(1H,3H)-diones of general formula I: 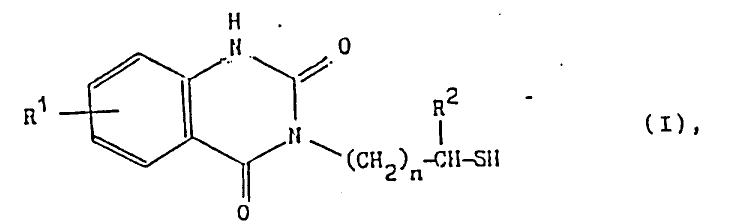 wherein:
R¹ is hydrogen, 6-methyl, 6-fluoro, 6-chloro, 6-bromo or 6,7-dimethoxy,
R² is hydrogen or methyl and
n is 1 or 2,
including their pharmaceutically acceptable salts and their tautometers.

2. 3-(Mercaptoalkyl)quinazoline-2,4(1H,3H)-diones according to claim 1 of general formula II: 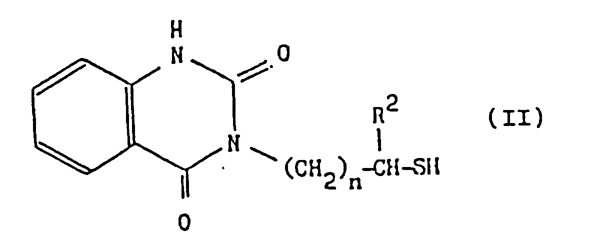 where R² and n are as in claim 1, including their pharmaceutically acceptable salts and their tautometers.

3. A process for the preparation of the compounds of general formulae I and II according to claims 1 and 2, characterised by the following procedures:
(A)
- reaction of 2H-3,1-benzoxazine-2,4(1H)-diones of general formula III: 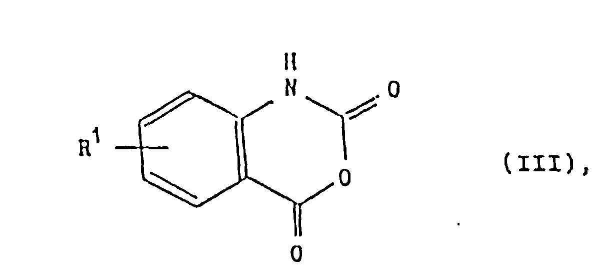 wherein R¹ is as defined in claim 1, with an amino alkanol of general formula IV: 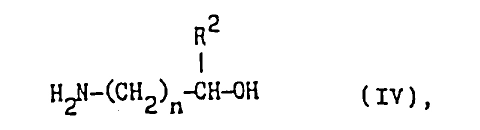 wherein R² and n are as defined in claim 1, to give a compound of general formula V: 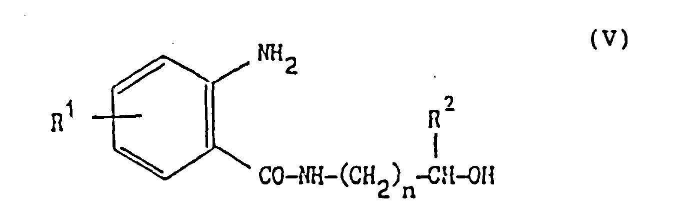 wherein R¹, R² and n are as defined above,
- addition of a reaction solution of a C₁₋₃-alkanol, carbon disulphide and sodium or potassium hydroxide or sodium or potassium xanthogenate,
- heating of the reaction mixture,
- cooling and acidification of the reaction mixture,
- heating of the resulting compounds of general formula VI: 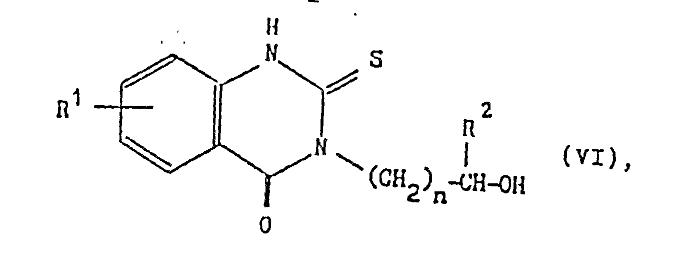 wherein R¹, R² and n are as defined above,
with a concentrated mineral acid such as hydrochloric acid and/or sulphuric acid, or mixtures of these mineral acids with glacial acetic acid and/or formic acid,
- and, if appropriate, subsequent addition of water to the reaction mixture and further heating under reflux to give the compounds of general formula I or their tautometers,
or
(B)
- heating of 4H-3,1-benzothiazine-2,4(1H)-dithiones of general formula VII: 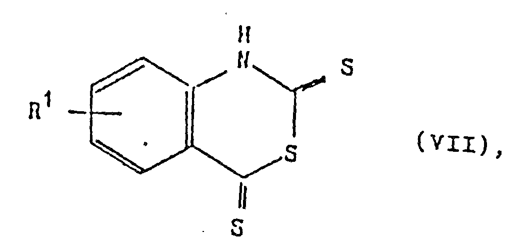 wherein R¹ is as defined above,
with an amino alkanol of general formula IV: 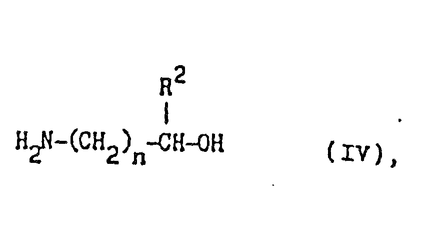 wherein R² and n are as defined above,
in a polar organic solvent to give the compounds of general formula VIII: 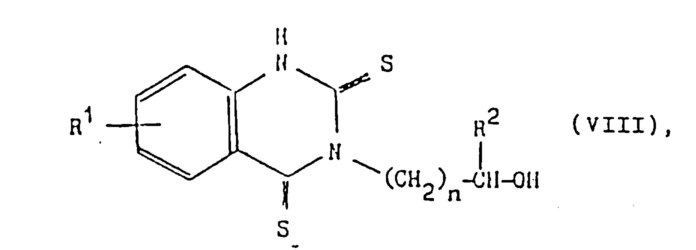 wherein R¹, R² and n are as defined above,
- reaction of the compounds of formula VIII, in an aqueous-alkanolic reaction medium, in the presence of an acid acceptor such as an alkali metal hydroxide or triethylamine, with an alkyl halide of general formula IX:
R³-X (IX),
wherein
R³ is C₁₋₃-alkyl and
x is iodine or bromine,
to give the compounds of general formula X: 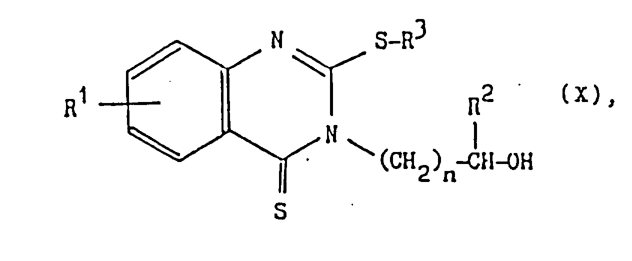 wherein R¹, R², R³ and n are as defined above,
- reaction of the compounds of formula X with alkanolic mineral acid to give tricyclic quinazolinium salts of general formula XI: 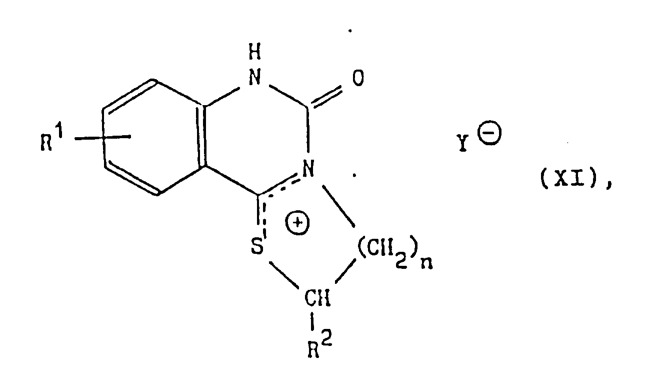 wherein R¹, R² and n are as defined above and Y is an acid anion, preferably chlorine,
- and conversion of the compounds of formula XI, by treatment in alkanolic-aqueous sodium hydroxide solution, followed by filtration and acidification of the filtrate with dilute mineral acid, to the compounds of general formula I or their tautometers,
or
(C)
- reaction of 2H-benzoxazine-2,4(1H)-diones of general formula III: 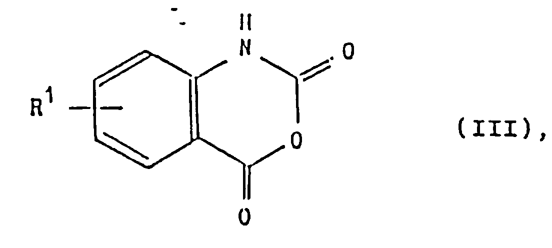 wherein R¹ is as defined above,
with bis(aminoalkyl)disulphanes of general formula XII: 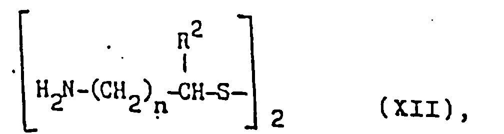 wherein R² and n are as defined above,
or their salts, in a solvent, to give compounds of general formula XIII: 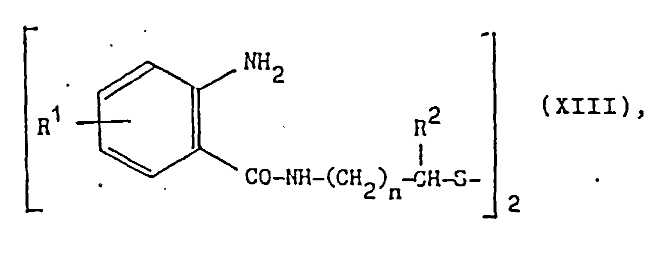 wherein R¹, R² and n are as defined above,
- reaction of the compounds of formula XIII with alkyl chloroformates of general formula XIV:
C1-COOR⁴ (XIV),
wherein R⁴ is C₁₋₃-alkyl,
in a solvent or solvent mixture, to give compounds of general formula XV: 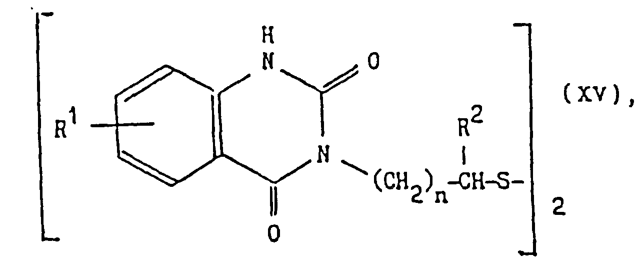 wherein R¹, R² and n are as defined above,
- and conversion of the compounds of formula XV, if appropriate after purification and drying, in a solvent or solvent mixture, with nascent hydrogen to the compounds of general formula I or their tautometers
or
(D)
- reaction of 3-(halogenoalkyl)-2,4-dioxo-1,2,3*-tetrahydroquinazolines of general formula XVI 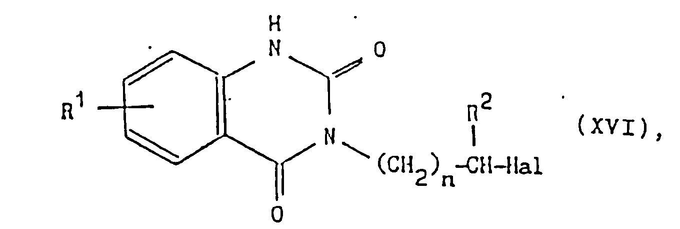 wherein R¹, R² and n are as defined above and Hal is chlorine or bromine,
with thiourea to give the S-[ω-(2,4-dioxo-1,2,3,4-tetrahydroquinazolin-3-yl)alkyl]isothi-uronium halides of general formula XVII: 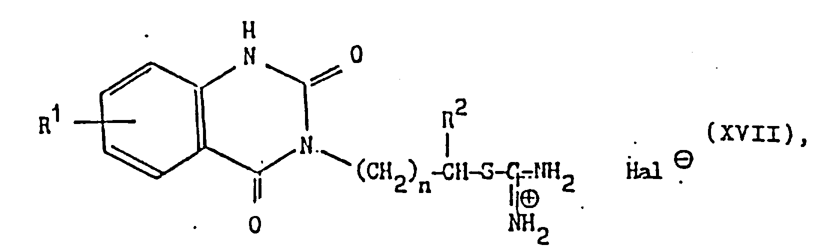 wherein R¹, R², n and Hal are as defined above,
- and conversion of the compounds of formula XVII, if appropriate after separation, with alkali metal hydroxide solution, to the compounds of general formula I or their tautometers,
and, if appropriate, conversion of the compounds of general formula I obtained by procedures A-D to corresponding salts such as the alkali metal salts or ammonium salts.

4. A process according to claim 3 characterised by one or more of the following measures:
(a) the reaction of the compounds of formula III with the compounds of formula IV is carried out in an aqueous reaction medium;
(b) the reaction of the compounds of formula VIII with the compounds of formula IX is carried out at room temperature;
(c) the conversion of the compounds of formula X to the compounds of formula XI is carried out with absolute ethanolic hydrochloric acid;
(d) the compounds of formula III are reacted according to variant (C) with the dihydrochlorides of the compounds of formula XII;
(e) the reaction of the compounds of formula XVI with thiourea is carried out in the temperature range 80 to 15°C;
(f) the conversion of the compounds of formula XVII to the compounds of formula I or their tautometers is carried out in the presence of an inert gas;
(g) the conversion of the compounds of formula XVII to the compounds of formula I or their tautometers is carried out in the temperature range -10 to +15°C.

5. A process according to claim 3(A) and/or 4 characterised in that the solid mixtures resulting from the reactions of the compounds of general formula VI with concentrated mineral acids such as hydrochloric acid or sulphuric acid, or mixtures of these mineral acids with glacial acetic acid and/or formic acid, are separated off and then converted to the compounds of general formula I or their tautometers by heating in dilute mineral acids.

6. A process according to claim 3(D) and/or 4 characterised in that the reaction of the compounds of general formula XVI with thiourea is carried out in a molar ratio of 1:1 to 1:3.

7. A process according to one or more of claims 3(D), 4 and 5 characterised in that, to prepare the compounds of general formula XVII, the reactants are reacted in a protic solvent under reflux and/or the reaction of unreactive compounds of general formula XVI is carried out between 100 and 150°C, if appropriate under vacuum.

8. A process according to claim 7 characterised in that, to prepare the compounds of general formula XVII, the reactants are reacted in methyl glycol or butan-1-ol.

9. A process according to one or more of claims 3(D) and 6 to 8 characterised in that the conversion of the compounds of general formula XVII to the compounds of general formula I is carried out with sodium hydroxide solution having a concentration of 0.1 to 1.0 mol/l, if appropriate with mechanical stirring or shaking.

10. A process according to claim 9 characterised in that the conversion of the compounds of general formula XVII to the compounds of general formula I is carried out with sodium hydroxide solution having a concentration of 0.25 mol/l.

11. Pharmaceutical formulations, characterised by one or more compounds of general formula I according to claim 1 and/or one or more compounds of general formula II according to claim 2, and inert, pharmaceutically acceptable adjuncts and/or excipients.

12. Pharmaceutical formulations, characterised by 3-(2-mercaptoethyl)quinazoline-2,4(1H,3H)-dione and/or its tautometer and/or pharmaceutically acceptable salts of these compounds as active substances.

13. A process for the preparation of the pharmaceutical formulations according to claims 11 and 12, characterised in that one or more compounds of general formula I according to claim 1 or one or more compounds of general formula II according to claim 2 are converted, with inert, pharmaceutically conventional adjuncts and/or excipients, in a manner known per se, to a form suitable for medicinal administration, such as tablets, coated tablets, capsules, suppositories, solutions or ampoules.

14. Use of the compounds of general formulae I and II according to claims 1 and 2, respectively, for the preparation of pharmaceutical formulations for the treatment of immune diseases, including the accomplishment of a stimulating and/or restoring action on the immune system.

15. Use of the compounds of general formulae I and II according to claims 1 and 2, respectively, for the preparation of pharmaceutical formulations for the treatment of viral infections, including the accomplishment of an antiviral action, in human and veterinary medicine.

## Revendications

1. 3-(mercaptoalkyl)-quinazoline-2,4(1H,3H)-diones de formule générale I 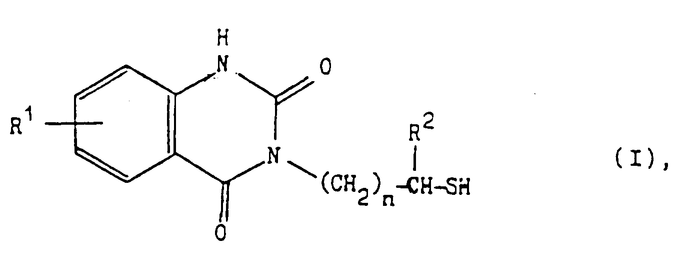 dans laquelle
R¹ représente l'hydrogène, un substituant 6-méthyle, 6-fluoro, 6-chloro, 6-bromo ou 6,7-diméthoxy,
R² représente l'hydrogène ou un groupe méthyle
et
n est égal à 1 ou 2,
y compris leurs sels convenant pour les usages pharmaceutiques et leurs tautomères.

2. 3-(mercaptoalkyl)-quinazoline-2,4(1H,3H)-diones selon la revendication 1, de formule générale II 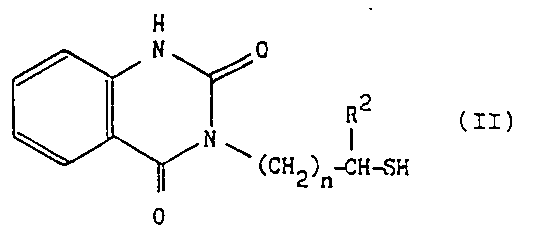 dans laquelle R² et n ont les significations indiquées dans la revendication 1,
y compris leurs sels convenant pour les usages pharmaceutiques et leurs tautomères.

3. Procédé de préparation de composés de formules générales I et II selon les revendications 1 et 2,
caractérisé par les modes opératoires suivants :
(A)
- réaction de 2H-3,1-benzoxazine-2,4(1H)-diones de formule générale III 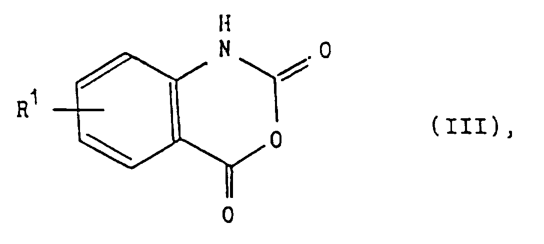 dans laquelle R¹ a les significations indiquées dans la revendication 1,
avec un aminoalcanol de formule générale IV 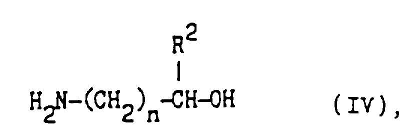 dans laquelle R² et n ont les significations indiquées dans la revendication 1,
ce qui donne un composé de formule générale V 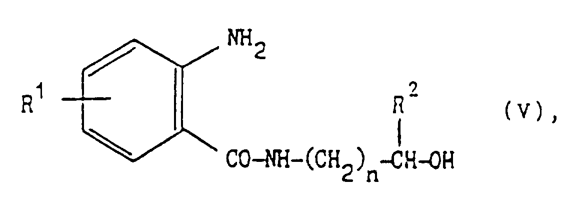 dans laquelle R¹, R² et n ont les significations déjà indiquées,
- addition d'une solution de réaction d'un alcanol en C₁-C₃, du sulfure de carbone et de l'hydroxyde de sodium ou de potassium ou de xanthogénate de sodium ou de potassium,
- chauffage du mélange de réaction,
- refroidissement et acidification du mélange de réaction,
- chauffage des composés obtenus, répondant à la formule générale VI 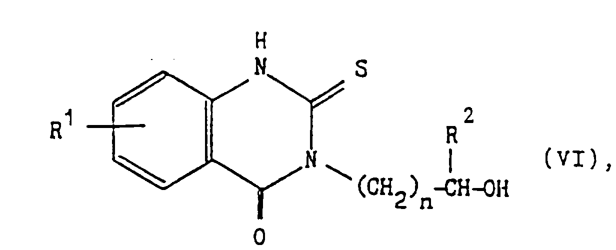 dans laquelle R¹, R² et n ont les significations indiquées ci-dessus,
avec un acide minéral concentré, par exemple l'acide chlorhydrique et/ou l'acide sulfurique, ou un mélange de tels acides minéraux avec l'acide acétique glacial et/ou l'acide formique,
- et le cas échéant, addition subséquente d'eau au mélange de réaction et nouveau chauffage au reflux, donnant les composés de formule générale I ou leurs tautomères,
ou bien
(B)
- chauffage de 4H-3,1-benzothiazine-2,4(1H)-dithiones de formule générale VII 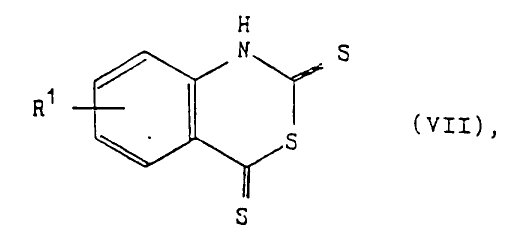 dans laquelle R¹ a les significations indiquées ci-dessus,
avec un aminoalcanol de formule générale IV 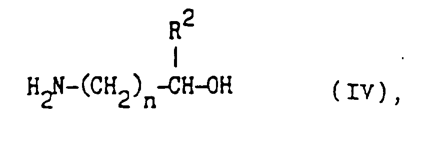 dans laquelle R² et n ont les significations indiquées ci-dessus,
dans un solvant organique polaire, donnant les composés de formule générale VIII 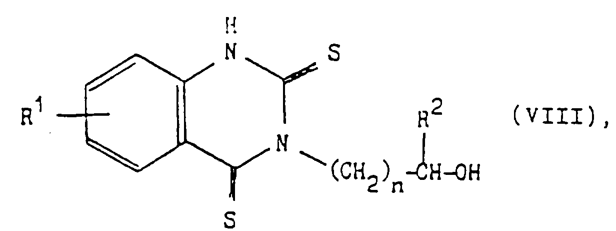 dans laquelle R¹, R² et n ont les significations indiquées ci-dessus,
- réaction des composés de formule VIII dans un milieu de réaction hydroalcanolique, en présence d'un accepteur d'acide tel qu'un hydroxyde alcalin ou la triéthylamine, avec un halogénure d'alkyle de formule générale IX
R³-X (IX),
dans laquelle R³ représente un groupe alkyle en C₁-C₃ et X l'iode ou le brome, donnant les composés de formule générale X 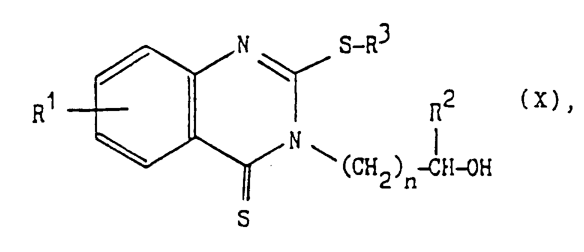 dans laquelle R¹, R², R³ et n ont les significations indiquées ci-dessus,
- réaction des composés de formule X avec un acide minéral alcanolique donnant les sels tricycliques de quinazolinium de formule générale XI 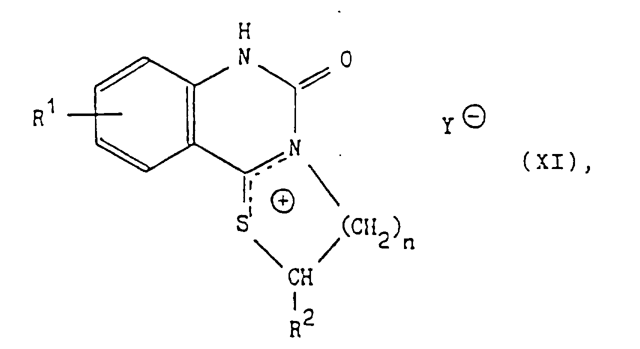 dans laquelle R¹, R² et n ont les significations indiquées ci-dessus et
Y représente l'anion d'un acide, de préférence le chlore,
et
- conversion des composés de formule XI, par traitement dans une lessive de soude aqueuse-alcanolique suivi d'une filtration et d'une acidification du filtrat à l'aide d'un acide minéral dilué, en les composés de formule générale I ou leurs tautomères,
ou bien
(C)
- réaction de 2H-benzoxazine-2,4(1H)-diones de formule générale III 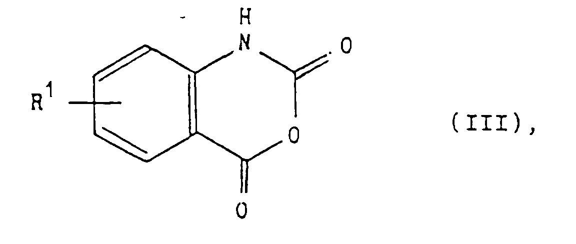 dans laquelle R¹ a les significations indiquées ci-dessus,
avec des bis-(aminoalkyl)disulfanes de formule générale XII 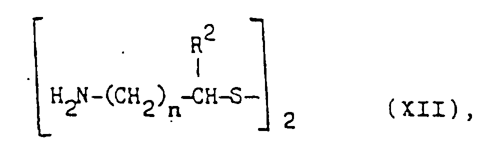 dans laquelle R² et n ont les significations indiquées ci-dessus,
ou leurs sels, dans un solvant, donnant les composés de formule générale XIII 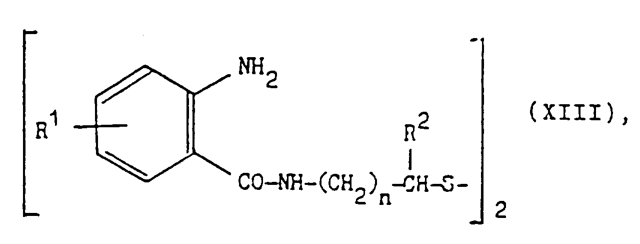 dans laquelle R¹, R² et n ont les significations indiquées ci-dessus,
- réaction des composés de formule XIII avec des chloroformiates d'alkyle de formule générale XIV
Cl-COOR⁴ (XIV),
dans laquelle R⁴ représente un groupe alkyle en C₁-C₃,
dans un solvant ou mélange solvant, donnant les composés de formule générale XV 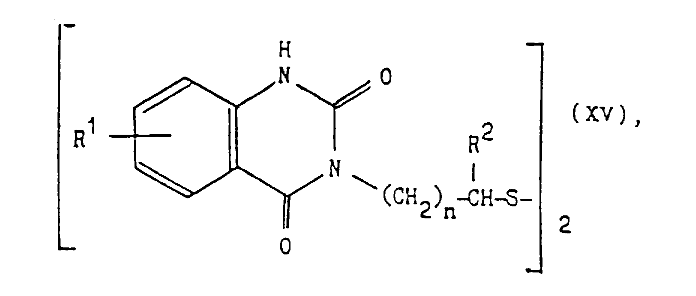 dans laquelle R¹, R² et n ont les significations indiquées ci-dessus,
et
- conversion des composés de formule XV, le cas échéant après purification et séchage, dans un solvant ou mélange solvant, à l'aide de l'hydrogène naissant, en les composés de formule générale I ou leurs tautomères,
ou bien
(D)
- réaction de 3-(halogénoalkyl)-2,4-dioxo-1,2,3-tétrahydroquinazolines de formule générale XVI 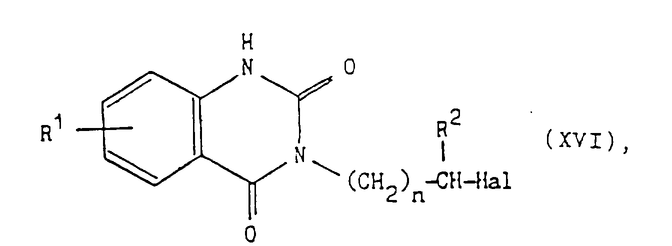 dans laquelle R¹, R² et n ont les significations indiquées ci-dessus et
Hal représente le chlore ou le brome,
avec la thiourée, donnant les halogénures de S-[ω-2,4-dioxo-1,2,3,4-tétrahydroquinazoline-3-yl)-alkyl]-7-isothiuronium de formule générale XVII 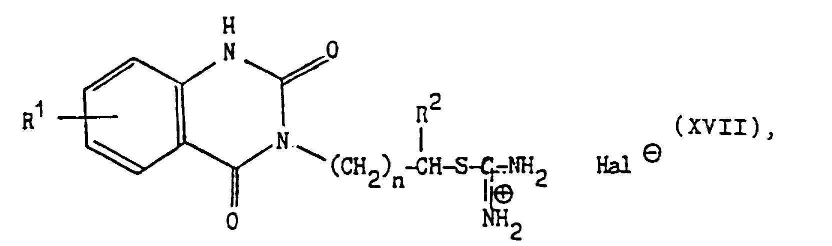 dans laquelle R¹, R², n et Hal ont les significations indiquées ci-dessus,
et
- conversion des composés de formule XVII, éventuellement après leur séparation, et à l'aide d'une lessive alcaline, en les composés de formule générale I ou leurs tautomères,
et le cas échéant, conversion des composés de formule générale I obtenus par les modes opératoires A à D en les sels correspondants tels que les sels alcalins ou d'ammonium.

4. Procédé selon la revendication 3, caractérisé par une ou plusieurs des dispositions suivantes :
(a) la réaction de composés de formule III avec les composés de formule IV est réalisée dans un milieu de réaction aqueux ;
(b) la réaction des composés de formule VIII avec les composés de formule IX est réalisée à température ambiante ;
(c) la conversion des composés de formule X en les composés de formule XI est réalisée à l'aide d'acide chlorhydrique éthanolique anhydre ;
(d) les composés de formule III sont mis à réagir selon la variante (C) avec les dichlorhydrates des composés de formule XII ;
(e) la réaction des composés de formule XVI avec la thiourée est réalisée dans l'intervalle de température de 80 à 150°C ;
(f) la conversion des composés de formule XVII en les composés de formule I ou leurs tautomères est réalisée en présence d'un gaz protecteur ;
(g) la conversion des composés de formule XVII en les composés de formule I ou leurs tautomères est réalisée dans l'intervalle de température de -10 à +15°C.

5. Procédé selon les revendications 3(A) et/ou 4, caractérisé en ce que l'on sépare les mélanges de substances solides obtenus dans les réactions des composés de formule générale VI avec des acides minéraux concentrés tels que l'acide chlorhydrique ou l'acide sulfurique, ou avec des mélanges de tels acides minéraux avec l'acide acétique glacial et/ou l'acide formique et on les convertit ensuite par chauffage dans des acides minéraux dilués en les composés de formule générale I ou leurs tautomères.

6. Procédé selon les revendications 3(D) et/ou 4, caractérisé en ce que la réaction entre les composés de formule générale XVI et la thiourée est réalisée à un rapport molaire de 1:1 à 1:3.

7. Procédé selon une ou plusieurs des revendications 3(D), 4 et 5, caractérisé en ce que, pour la préparation des composés de formule générale XVII, on fait réagir les composants dans un solvant protonique en chauffant au reflux et/ou on procède à la conversion des composés de formule générale XVI qui sont peu réactifs à des températures de 100 à 150°C, éventuellement sous vide.

8. Procédé selon la revendication 7, caractérisé en ce que, pour la préparation des composés de formule générale XVII, on fait réagir les composants dans le méthylglycol ou le butane-1-ol.

9. Procédé selon une ou plusieurs des revendications 3(D) et 6 à 8, caractérisé en ce que la conversion des composés de formule générale XVII en les composés de formule générale I est réalisée à l'aide d'une lessive de soude à une concentration de 0,1 à 1,0 mol/l, le cas échéant sous agitation mécanique ou sous secousses.

10. Procédé selon la revendication 9, caractérisé en ce que la conversion des composés de formule générale XVII en les composés de formule générale I est réalisée à l'aide d'une lessive de soude à une concentration de 0,25 mol/l.

11. Compositions pharmaceutiques, caractérisées en ce qu'elles contiennent un ou plusieurs composés de formule générale I selon la revendication 1 et/ou un ou plusieurs composés de formule générale II selon la revendication 2, avec des produits auxiliaires et/ou véhicules inertes convenant pour l'usage pharmaceutique.

12. Compositions pharmaceutiques, caractérisées en ce qu'elles contiennent en tant que substances actives la 3-(2-mercaptoéthyl)-quinazoline-2,4(1H,3H)-dione et/ou son tautomère et/ou des sels de ces composés convenant pour l'usage pharmaceutique.

13. Procédé de préparation des compositions pharmaceutiques selon les revendications 11 et 12, caractérisé en ce que l'on met, de manière connue en soi, un ou plusieurs composés de formule générale I selon la revendication 1 ou un ou plusieurs composés de formule générale II selon la revendication 2 sous une forme appropriée à l'administration médicinale, par exemple sous la forme de comprimés, de dragées, de capsules, de suppositoires, de solutions ou d'ampoules, avec des produits auxiliaires et/ou véhicules inertes usuels en pharmacie.

14. Utilisation des composés de formules générales I et II selon les revendications respectives 1 et 2 pour la préparation de compositions pharmaceutiques prévues pour le traitement des maladies immunitaires, y compris en vue d'un effet de stimulation de l'immunité et/ou de rétablissement de l'immunité.

15. Utilisation des composés de formules générales I et II des revendications respectives 1 et 2 pour la préparation de compositions pharmaceutiques prévues pour le traitement des infections virales, y compris en vue d'une activité antivirale, en médecine humaine et vétérinaire.
